(19) Europäisches Patentamt
European Patent Office
Office européen des brevets

(11) EP 1 512 004 B1

(12) **EUROPEAN PATENT SPECIFICATION**

(45) Date of publication and mention of the grant of the patent:
**15.03.2006 Bulletin 2006/11**

(21) Application number: **03740219.5**

(22) Date of filing: **11.06.2003**

(51) Int Cl.:
***G01N 33/50*** *(2006.01)*

(86) International application number:
**PCT/EP2003/006110**

(87) International publication number:
**WO 2003/104803 (18.12.2003 Gazette 2003/51)**

(54) **METHOD FOR MAPPING AND ELIMINATING T-CELL EPITOPES**

METHODE ZUR IDENTIFIZIERUNG UND ELIMINIERUNG VON T-ZELL EPITOPEN

METHODE DE CARTOGRAPHIE ET D'ELIMINATION DES EPITOPES T

(84) Designated Contracting States:
**AT BE BG CH CY CZ DE DK EE ES FI FR GB GR HU IE IT LI LU MC NL PT RO SE SI SK TR**

(30) Priority: **11.06.2002 EP 02012919**

(43) Date of publication of application:
**09.03.2005 Bulletin 2005/10**

(73) Proprietor: **Merck Patent GmbH**
**64293 Darmstadt (DE)**

(72) Inventors:
- **BAKER, Matthew**
  **Ely,**
  **Cambridge CB6 1AR (GB)**
- **CARR, Francis J.**
  **Balmedie,**
  **Aberdeenshire AB23 8XU (GB)**
- **CARTER, Graham**
  **By Newmachar,**
  **Aberdeenshire AB21 7XB (GB)**

(56) References cited:
**WO-A-02/40997** **GB-A- 2 349 463**

- **REECE JEANETTE C ET AL: "Mapping the major human T helper epitopes of tetanus toxin. The emerging picture" JOURNAL OF IMMUNOLOGY, vol. 151, no. 11, 1993, pages 6175-6184, XP002269741 ISSN: 0022-1767 cited in the application**
- **HIEMSTRA HOEBERT S ET AL: "The identification of CD4+ T cell epitopes with dedicated synthetic peptide libraries" PROCEEDINGS OF THE NATIONAL ACADEMY OF SCIENCES OF THE UNITED STATES, vol. 94, no. 19, 1997, pages 10313-10318, XP002269742 1997 ISSN: 0027-8424 cited in the application**
- **LAUNOIS PASCAL ET AL: "T-cell-epitope mapping of the major secreted mycobacterial antigen Ag85A in tuberculosis and leprosy" INFECTION AND IMMUNITY, vol. 62, no. 9, 1994, pages 3679-3687, XP000572211 ISSN: 0019-9567**
- **WARMERDAM PETRA A M ET AL: "Staphylokinase-specific cell-mediated immunity in humans." JOURNAL OF IMMUNOLOGY, vol. 168, no. 1, 1 January 2002 (2002-01-01), pages 155-161, XP002269743 ISSN: 0022-1767 (ISSN print) cited in the application**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 512 004 B1

**Description**

FIELD OF THE INVENTION

The present invention relates to the field of immunology. The invention provides screening methods for the identification of determinants and epitopes on protein molecules able to evoke an immune response. In particular the invention is concerned with the identification of epitopes for T-cells in therapeutic proteins. Finally, the invention relates to a combined approach of using epitope mapping in concert with the identification of MHC class II ligands deriving from said epitope mapping method and design of sequence analogous having a reduced number of such ligands and epitopes, respectively.

BACKGROUND OF THE INVENTION

There are many instances whereby the efficacy of a therapeutic protein is limited by an unwanted immune reaction to the therapeutic protein. Several mouse monoclonal antibodies have shown promise as therapies in a number of human disease settings but in certain cases have failed due to the induction of significant degrees of a human anti-murine antibody (MAMA) response [Schroff, R. W. et al (1985) *Cancer Res.* 45: 879-885; Shawler, D.L. et al (1985) *J. Immunol.* 135: 1530-1535]. For monoclonal antibodies, a number of techniques have been developed in attempt to reduce the HAMA response [WO 89/09622; EP 0239400; EP 0438310; WO 91/06667]. These recombinant DNA approaches have generally reduced the mouse genetic information in the final antibody construct whilst increasing the human genetic information in the final construct. Notwithstanding, the resultant "humanized" antibodies have, in several cases, still elicited an immune response in patients [Issacs J.D. (1990) *Sem. Immunol.* 2: 449, 456; Rebello, P.R. et al (1999) *Transplantation* 68: 1417-1420].

Antibodies are not the only class of polypeptide molecule administered as a therapeutic agent against which an immune response may be mounted. Even proteins of human origin and with the same amino acid sequences as occur within humans can still induce an immune response in humans. Notable examples amongst others include the therapeutic use of granulocyte-macrophage colony stimulating factor [Wadhwa, M. et al (1999) *Clin. Cancer Res.* 5: 1353-1361] and interferon alpha 2 [Russo, D. et al (1996) *Bri. J. Haem.* 94: 300-305; Stein, R. et al (1988) *New Engl. J. Med.* 318: 1409-1413]. In such situations where these human proteins are immunogenic, there is a presumed breakage of immunological tolerance that would otherwise have been operating in these subjects to these proteins.

This situation is different where the human protein is being administered as a replacement therapy for example in a genetic disease where there is a constitutional lack of the protein such as can be the case for diseases such as hemophilia A, Christmas disease, Gauchers disease and numerous other examples. In such cases, the therapeutic replacement protein may function immunologically as a foreign molecule from the outset, and where the individuals are able to mount an immune response to the therapeutic, the efficacy of the therapy is likely to be significantly compromised.

Irrespective of whether the protein therapeutic is seen by the host immune system as a foreign molecule, or if an existing tolerance to the molecule is overcome, the mechanism of immune reactivity to the protein is the same. Key to the induction of an immune response is the presence within the protein of peptides that can stimulate the activity of T-cells via presentation on MHC class II molecules, so-called "T-cell epitopes". Such T-cell epitopes are commonly defined as any amino acid residue sequence with the ability to bind to MHC Class II molecules. Implicitly, a "T-cell epitope" means an epitope which when bound to MHC molecules can be recognized by a T-cell receptor (TCR), and which can, at least in principle, cause the activation of these T-cells by engaging a TCR to promote a T -cell response.

MHC Class II molecules are a group of highly polymorphic proteins that play a central role in helper T-cell selection and activation. The human leukocyte antigen group DR (HLA-DR) are the predominant isotype of this group of proteins however, isotypes HLA-DQ and HLA-DP perform similar functions. The present invention is applicable to the detection of T-cell epitopes presented within the context of DR, DP or DQ MHC Class II. In the human population, individuals bear two to four DR alleles, two DQ and two DP alleles. The structure of a number of DR molecules has been solved and these appear as an open-ended peptide binding groove with a number of hydrophobic pockets which engage hydrophobic residues (pocket residues) of the peptide [Brown et al *Nature* (1993) 364: 33; Stem et al (1994) *Nature* 368: 215]. Polymorphism identifying the different allotypes of class II molecule contributes to a wide diversity of different binding surfaces for peptides within the peptide binding groove and at the population level ensures maximal flexibility with regard to the ability to recognize foreign proteins and mount an immune response to pathogenic organisms.

An immune response to a therapeutic protein proceeds via the MHC class II peptide presentation pathway. Here exogenous proteins are engulfed and processed for presentation in association with MHC class II molecules of the DR, DQ or DP type. MHC Class II molecules are expressed by professional antigen presenting cells (APCs), such as macrophages and dendritic cells amongst others. Engagement of a MHC class II peptide complex by a cognate T-cell receptor on the surface of the T-cell, together with the cross-binding of certain other co-receptors such as the CD4 molecule, can induce an activated state within the T-cell. Activation leads to the release of cytokines further activating

other lymphocytes such as B cells to produce antibodies or activating T killer cells as a full cellular immune response.

T-cell epitope identification is the first step to epitope elimination, however there are few clear cases in the art where epitope identification and epitope removal are integrated into a single scheme. Thus WO98/52976 and WO00/34317 teach computational threading approaches to identifying polypeptide sequences with the potential to bind a sub-set of human MHC class II DR allotypes. In these teachings, predicted T-cell epitopes are removed by the use of judicious amino acid substitution within the protein of interest. However with this scheme and other computationally based procedures for epitope identification [Godkin, A.J. et al (1998) *J. Immunol.* 161: 850-858; Sturniolo, T. et al (1999) *Nat. Biotechnol.* 17: 555-561], peptides predicted to be able to bind MHC class II molecules may not function as T-cell epitopes in all situations, particularly, *in vivo* due to the processing pathways or other phenomena. In addition, the computational approaches to T-cell epitope prediction have in general not been capable of predicting epitopes with DP or DQ restriction.

Equally, *in vitro* methods for measuring the ability of synthetic peptides to bind MHC class II molecules, for example using B-cell lines of defined MHC allotype as a source of MHC class II binding surface [Marshall K.W. et al. (1994) *J. Immunol.* 152:4946-4956; O'Sullivan et al (1990) *J. Immunol.* 145: 1799-1808; Robadey C. et al (1997) *J. Immunol* 159: 3238-3246], may be applied to MHC class II ligand identification. However, such techniques are not adapted for the screening multiple potential epitopes to a wide diversity of MHC allotypes, nor can they confirm the ability of a binding peptide to function as a T-cell epitope.

Recently techniques exploiting soluble complexes of recombinant MHC molecules in combination with synthetic peptides have come into use [Kern, F. et al (1998) *Nature Medicine* 4:975-978; Kwok, W.W. et al (2001) *TRENDS in Immunol.* 22:583-588]. These reagents and procedures are used to identify the presence of T-cell clones from peripheral blood samples from human or experimental animal subjects that are able to bind particular MHC-peptide complexes and are not adapted for the screening multiple potential epitopes to a wide diversity of MHC allotypes.

Biological assays of T-cell activation remain the best practical option to providing a reading of the ability of a test peptide/protein sequence to evoke an immune response. Examples of this kind of approach include the work of Petra et al using T-cell proliferation assays to the bacterial protein staphylokinase, followed by epitope mapping using synthetic peptides to stimulate T-cell lines [Petra, A.M. et al (2002) *J. Immunol.* 168: 155-161]. Similarly, T-cell proliferation assays using synthetic peptides of the tetanus toxin protein have resulted in definition of immunodominant epitope regions of the toxin [Reece J.C. et al (1993) *J. Immunol.* 151: 6175-6184]. WO99/53038 discloses an approach whereby T-cell epitopes in a test protein may be determined using isolated sub-sets of human immune cells, promoting their differentiation *in vitro* and culture of the cells in the presence of synthetic peptides of interest and measurement of any induced proliferation in the cultured T-cells. The same technique is also described by Stickler et al [Stickler, M.M. et al (2000) *J. Immunotherapy* 23:654-660], where in both instances the method is applied to the detection of T-cell epitopes within bacterial subtilisin. Such a technique requires careful application of cell isolation techniques and cell culture with multiple cytokine supplements to obtain the desired immune cell sub-sets (dendritic cells, CD4+ and or CD8+ T-cells) and is not conducive to rapid through-put screening using multiple donor samples.

In a variation of these approaches, Hiemstra et al [Hiemstra, H.S. (1997) *Proc. Natl. Acad. Sci USA* 94: 10313-10318] have described a procedure for identifying a peptide epitope capable of stimulating a known T-cell, such a process is valuable in the detection of autoreactive T-cell clones for which the (auto)antigen is unknown.

The above examples and other biological assays involving technical variations on the theme of measuring an *in vitro* T-cell activation event, usually by the measurement of an induced proliferation response, abound. However, none of the procedures provide a unified scheme for the detection of biologically relevant epitopes in proteins of human origin nor are readily applicable to the detection of epitopes of significance to a wide population of MHC allotypes. The present invention is conceived to provide such a scheme and provides a basis for the identification and removal of T-cell epitopes from a given in principal therapeutically valuable but originally immunogenic peptide, polypeptide or protein.

In summary the invention relates to the following issues:

- Using a panel of synthetic peptides in a naïve T-cell assay to map the immunogenic region(s) of a protein therapeutic, and in particular a protein therapeutic whereby the protein is a human protein;
- using a panel of synthetic peptides in a recall assay to fine map the immunogenic region(s) of a protein therapeutic.
- using a panel of whole protein variants in a naive T-cell assay to select variants displaying minimal immunogenicity *in vitro;*
- using a panel of synthetic peptide variants in a naive T-cell assay to select peptide sequences displaying minimal immunogenicity *in vitro;*
- using biological assays of T-cell stimulation to select a peptide sequence which exhibits a stimulation index of less than 2.0 and preferably less than 1.8 in a naive T-cell assay;
- using biological assays of T-cell stimulation to select a protein variant which exhibits a stimulation index of less than 2.0 and preferably less than 1.8 in a naïve T-cell assay;
- a strategy in which T-cell lines are developed from individuals previously in receipt of a protein therapeutic and use

of those cell lines to map the immunogenic region(s) of the therapeutic molecule;

- a strategy according to the above in which in addition B-cell lines are developed in parallel to the T-cell lines developed from individuals previously in receipt of a protein therapeutic and the combined use of the those lines to map the immunogenic region(s) of the therapeutic molecule;
- use of B-cell lines developed from individuals previously in receipt of a protein therapeutic and in parallel to the development of T-cell lines from the same individuals as a source of autologous APC in further rounds of T-cell stimulation or optionally as a binding surface for synthetic peptide binding assays;
- *construction* of a *T-cell* epitope map of a subject protein using PBMC isolated from healthy donors and a screening method involving the steps comprising:

  i) antigen priming *in vitro* using synthetic peptide or whole protein immunogen for a culture period of up to 7 days;
  ii) addition of IL-2 and culture for up to 3 days;
  iii) addition of primed T cells to autologous irradiated PBMC and re-challenge with antigen for a further culture period of 4 days and
  iv) measurement of T cell activation e.g, proliferation index by any suitable method;

- construction of a T-cell epitope map of a subject protein using PBMC isolated from patients in whom there exists an established immune response to the subject protein or derivatives thereof and application of a screening method involving the steps comprising:

  i) - antigen priming *in vitro* using synthetic peptide or whole protein immunogen for a culture period of up to 7 days, ii)
  ii) addition of IL-2 and culture for up to 3 days, iii)
  iii) addition of primed T cells to autologous irradiated PBMC and re-challenge with antigen for a further culture period of up to 4 days and
  iv) measurement of T cell activation e.g. proliferation index by any suitable method;

- construction of a T-cell epitope map exploiting polyclonal or monoclonal cell lines derived from PBMC samples from healthy donors or patients with established immune responses to a protein of interest. Developing said cell lines into an immunologically primed state by one or multiple rounds of a priming step culminating in expansion of cell numbers in the presence of IL-2 +/phytohaemagglutinin (PHA) or other mitogenic stimulus. Said primed cells are contacted with either individual synthetic peptides or peptide pools comprising multiple synthetic peptides and lines stimulated into proliferation detected using any suitable means. Where stimulation is detected from a pooled peptide immunogen the identity of the stimulating peptide is uncovered using further round of screening with individual peptides or smaller peptide pools and further primed cells;
- a concerted method for mapping the location of T-cell epitopes in protein sequences using naive T-cell activation assays and a computational scheme simulating the binding of the peptide ligand with one or more MHC allotypes;
- a method for locating T-cell epitopes in protein sequences comprising the following steps;

  i) use of naïve T-cell activation assays and synthetic peptides collectively encompassing the protein sequence of interest to identify epitope regions capable of activating T-cells;
  ii) use of a computational scheme simulating the binding of the peptide ligand with one or more MHC allotypes to analyse the epitope regions identified in step (i) and thereby identify MHC class II ligands within the epiaope region;
  iii) use of a computational scheme simulating the binding of the peptide ligand with one or more MHC allotypes to identify sequence analogues of the MHC ligands encompassed within the epitope region(s) which no longer bind MHC class II or bind with lowered affinity to a lesser number of MHC allotypes;
  iv) use of naive T-cell activation assays and synthetic peptides encompassing entirely or in collection encompassing the epitope regions identified within the protein of interest and testing the sequence analogues in naive T-cell activation assay in parallel with the wild-type (parental) sequences;

- a method according to the above scheme wherein steps (ii) and (iii) are carried out using a computational approach as taught by WO 02/069232;
- a method according to the above scheme where the naïve T-cell activation assay is conducted using PBMC cells derived from around 20 or more unrelated donors;
- a method according to the above scheme where the location of a T-cell epitope is found when a stimulation index score of around 2.0 is observed in two or more independent donor samples;
- a method according to the above scheme where the location of a T-cell epitope is found when a stimulation index

score of around 2.0 is observed in two or more independent donor samples and where one or more MHC class II ligands can be identified within the same sequence locale using a computational system;

- a method according to the above scheme whereby the computational system is according to the method as taught by WO 02/069232;
- identification of protein sequences with reduced ability to promote an immune response may be achieved using immunologically primed cells of the aforementioned scheme and a screening process whereby multiple variant peptides or whole protein antigens are tested in parallel to reference peptide pools or whole protein antigen containing only wild-type sequences. Peptides or protein variants with a lesser stimulation index to reference pools or wild-type protein are selected for further analysis;
- identification of protein sequences or protein preparations with increased ability to promote an immune response achieved using immunologically primed cells of the aforementioned scheme and a screening process whereby one or more peptides or whole protein antigens are tested in parallel to reference peptide pools or whole protein antigen giving a known *in vitro* immune response. Peptides or protein preparations shown to evoke a different stimulation index profile to the reference preparations are selected for further analysis or may be eliminated from the production process;
- peptide sequences able to evoke a stimulation index of greater than 1.8 and preferably greater than 2.0 in a naïve T-cell assay and selected from any therapeutic protein;
- peptide sequences selected from any therapeutic protein having a stimulation index of greater than 1.8 and preferably greater than 2.0 in a naïve T-cell assay wherein the peptide is modified to a minimum extent and tested in the naive T-cell assay and found to have a stimulation index of less than 2.0;
- peptide sequences sharing 100% amino acid identity with the wild-type protein sequence and able to evoke a stimulation index of 1.8 or greater and preferably greater than 2.0 in a T-cell assay;
- a protein molecule in which the immunogenic regions have been mapped using a T-cell assay and then modified such that upon re-testing in a T-cell assay the modified protein evokes a stimulation index smaller than the parental (non-modified) molecule and most preferably less than 2.0

DETAILED DESCRIPTION OF THE INVENTION

According to the first embodiment of the invention there is provided a method whereby a protein antigen may be screened for the presence of determinants within its sequence capable of evoking a T-cell driven immune response should that protein be introduced in its un-modified state into a human subject. The method thereby provides a predictive tool for the identification of T-cell epitopes in proteins with therapeutic potential in man where the protein is to be provided for the therapy of an acquired disease state and where that protein may be a human protein.

It is particularly desired to provide an epitope map of a protein of interest where the map has relevance to a wide spectrum of possible MHC allotypes. It is desired that the map is sufficiently representative to allow the design or selection of a modified protein for which the ability of the protein to evoke a T-cell driven immune response is eliminated or at least ameliorated for the majority of patients to whom the protein is likely to be administered. Accordingly in the practice of the invention the screening process exploiting PBMC derived T-cells from naïve donors is collected from a pool of donors of sufficient immunological diversity to provide a sample of at least greater than 90% of the MHC class II repertoire (HLA-DR) extant in the human population and preferably greater than 95% of that repertoire. In an ideal situation, equivalence to greater than 99% representation is preferred, although it is recognised that there are practical limitations to achieving this ideal. Accordingly, where a naïve T-cell response is to be detected to a given synthetic peptide, the peptide in practice will be contacted with PBMC preparations derived from multiple donors in isolation, the numbers of donors or herein more preferably described as the "donor pool", is for practical purposes not likely to be less than 20 unrelated individuals (pre-selected according to their MHC class II haplotypes).

The term "naive donor" in the context of the present invention means that the T-cells obtained from the individual have not previously been exposed to the protein or peptide antigen of interest, and where the protein antigen is a human protein, the individual has not been in receipt of any therapeutic or exogenous sources of the protein.

Thus according to the first embodiment of the present, there is provided a method for T-cell epitope mapping exploiting immunologically naïve T-cells. The T-cells are provided from a peripheral blood sample from a multiplicity of different healthy donors for whom the protein of interest may be an endogenous molecule but who have not been in receipt of the protein of interest from any exogenous source e.g. administered therapeutically. The assay is conducted using PBMC cultured *in vitro* using procedures common in the art and involves contacting the PBMC with synthetic peptide species representative of the protein of interest, and following a suitable period of incubation, measurement of peptide induced T cell activation such as cellular proliferation. Measurement is by any suitable means and may for example be conducted using $^{3}H$-thymidine incorporation whereby the accumulation of $^{3}H$ into cellular material is readily measured instrumentally. The degree of cellular proliferation for each combination of PBMC sample and synthetic peptide is examined relative to that seen in non peptide treated PBMC sample. Reference may also be made to the proliferative

response seen following treatment with a peptide or peptides for which there is an expected proliferative effect. In this regard is considered particularly advantageous to use peptide with known broad MHC restriction and especially peptide epitopes with MHC restriction to the DP or DQ isotypes.

To facilitate assembly of an epitope map for a given protein of interest, a set of synthetic peptides representative of the sequence of the protein are produced. A typical analysis under the scheme of the present involves the use of peptides containing 15 amino acid residues although it will be recognised that a peptide containing not less than 9 amino acid residues is in principle a suitable peptide. Peptides significantly exceeding 15 amino acid residues may also be used but it will equally be recognised that possible secondary structural effects or complexities of intracellular processing may obscure the ability of the peptide to induce a proliferative response. In order to scan the entire length of given protein, a particularly convenient scheme is to produce synthetic peptides each of 15 amino acid residues in length and each overlapping the next peptide in the series by 12 amino acid residues, i.e. each successive peptide in the series incrementally adds a further 3 amino acids to the analysis. In this way any given adjacent pair of peptides will map 18 amino acids of contiguous sequence in the protein of interest. Thus for a protein of interest comprising n amino acid residues, the number of 15-mer synthetic peptides required for a complete scan of the said protein will be $1+(n-12)/3$. Other schemes may be contemplated and be equally efficacious.

Using the scheme outlined above and exemplified in detail within the EXAMPLES herein, the inventors have discovered regions of protein sequence capable of evoking a proliferative response in naïve PBMC from different individual healthy donors. The protein sequences in question are sequence strings derived from whole human proteins for which there could be an expectation of immune tolerance but which none the less there is a demonstrable ability to evoke a surrogate immune response *in vitro.* This ability by extension may also apply *in vivo* should either of the proteins in question be administered for example as therapeutic entities. Specifically these proteins are interferon $\alpha$2 and interferon B. Both of these proteins are used therapeutically and significantly for both of these molecules immunogenic responses to these molecules in patients have been recorded [Russo, D. et al (1996) *ibid;* Stein, R. et al (1988) *ibid;* Myhr, K.M. et al (2000) Neurology 55:1569-1572; Bertolotto, A. et al (2000) *Immunopharmacology* 48: 95-100]. The present invention therefore provides a generalised scheme for the elucidation of epitope regions within normal human proteins and demonstrates the ability of peptides derived from these proteins to evoke an *in vitro* proliferative response in naïve PBMC derived from healthy donors.

A particularly effective method for defining a T-cell map using naïve T-cell assays of the first embodiment is provided in the EXAMPLES 1 and 2 whereby immunogenic regions of the molecules interferon beta (IFN$\beta$) and interferon alpha 2 (IFN$\alpha$2) are disclosed. A particularly preferred method for the identification of T-cell epitopes in proteins which are weakly immunogenic *in vivo* is described in EXAMPLE 3.

In a second embodiment where the invention provides for the elucidation of a T-cell epitope map, such a map may be used to guide the design of a modified protein whereby the epitope regions on the molecule are suitably modified such that they are no longer able to evoke a proliferative response according to the scheme of the invention and the protein of interest is thereby rendered less immunogenic to man.

According to this second embodiment, suitable modifications to the protein may include amino acid substitution of particular residues or combinations of residues. For the elimination of T-cell epitopes, amino acid substitutions are preferably made at appropriate points within the peptide sequence predicted to achieve substantial reduction or elimination of the activity of the T-cell epitope. In practice an appropriate point will preferably equate to an amino acid residue binding within one of the pockets provided within the MHC class II binding groove. It is most preferred to alter binding within the first pocket of the cleft at the so-called "P1" or "P1 anchor" position of the peptide. The quality of binding interaction between the PI anchor residue of the peptide and the first pocket of the MHC class II binding groove is recognised as being a major determinant of overall binding affinity for the whole peptide. An appropriate substitution at this position of the peptide will be for a residue less readily accommodated within the pocket, for example, substitution to a more hydrophilic residue. Amino acid residues in the peptide at positions equating to binding within other pocket regions within the MHC binding cleft are also considered and fall under the scope of the present.

It is understood that single amino acid substitutions within a given potential T-cell epitope are the most preferred route by which the epitope may be eliminated. Combinations of substitution within a single epitope may be contemplated and for example can be particularly appropriate where individually defined epitopes are in overlap with each other. Moreover, amino acid substitutions either singly within a given epitope or in combination within a single epitope may be made at positions not equating to the "pocket residues" with respect to the MHC class II binding groove, but at any point within the peptide sequence. Substitutions may be made with reference to an homologous structure or structural method produced using *in silico* techniques known in the art and may be based on known structural features of the molecule. For example a change may be contemplated to restore structure or biological activity of the variant molecule. Such compensatory changes and changes may also include deletion or addition of particular amino acid residues from the polypeptide.

A particularly effective means of removing epitopes from protein molecules is the concerted use of the naive T-cell activation assay scheme as outlined herein together with an *in silico* tool developed according to the scheme

described in WO 02/069232 which is incorporated fully herein by reference.

The software simulates the process of antigen presentation at the level of the peptide MHC class II binding interaction to provide a binding score for any given peptide sequence. Such a score is determined for many of the predominant MHC class II allotypes extant in the population. As this scheme is able to test any peptide sequence, the consequences of amino acid substitutions additions or deletions with respect to the ability of a peptide to interact with a MHC class II binding groove can be predicted. Consequently new sequence compositions can be designed which contain reduced numbers of peptides able to interact with the MHC class II and thereby function as immmunogenic T-cell epitopes. Where the biological assay using any one given donor sample can assess binding to a maximum of 4 DR allotypes, the *in silico* process can test the same peptide sequence using >40 allotypes simultaneously. In practice this approach is able to direct the design of new sequence variants which are compromised in the their ability to interact with multiple MHC allotypes.

By way of an example of utility of the combined approach to epitiope identification and removal, the results of a programme involving the engineering of human interferon alpha (IFNα) are provided herein. The entire human IFNα sequence was rendered into a set of 51 15-mer peptides (listed within table 2 of EXAMPLE 2). The T-cell assay was able to define three immunogenic regions (termed R1, R2 and R3) within the molecule and the software system according to the scheme of WO 02/069232 was able to identify predicted MHC class II ligands within each of the epitopes R1 -R3. Moreover, the system was further able to identify amino acid substitutions within the epitopes which resulted in significant loss of binding affinity between the peptide sequence and essentially all of the MHC class II allotypes represented in the system. A panel of synthetic peptides were constucted encompassing the wild-type epitope regions and variant sequences thereof in which MHC class II binding was eliminated by amino acid substitution. The peptides were used in naive T-cell activation assays and the stimulation index determined for each peptide and donor PBMC sample combination. In all instances where a donor sample was found to be responsive to a wild-type peptide, the variant peptide was found not to activate T-cells (FIGURE 3).

A preferred embodiment of the present invention is to use a modified T cell activation assay in which measurement of a T cell response is performed at different times after adding a test protein or peptide. This novel format for the assay is especially useful for detecting T cell responses in whole proteins or weakly immunogenic polypeptides. The assay format counteracts the complexity of components within the T cell assay mixture comprising a mixture of leukocytes and different molecules including cytokines. For any test protein or peptide, the kinetics of a T cell response in the assay is dependant on a number of factors including the status of T cells within the T cell assay mixture (for example, naive versus memory T cells), the concentration of cytokines at various timepoints, and the rate of generating significant T cell proliferation due to factors such as the concentration of specific peptide-MHC class II complexes. For any given protein or peptide, the peak of T cell proliferation in the assay system may peak before or after day 7 after addition of protein or peptide to, the assay mixture such that, by day 7 (the standard assay timepoint), T cell proliferation is not significant. By testing for T cell proliferation over a timecourse, for example on each of days 4, 5, 6, 7, 8 and 9, then T cell responses can be detected which would not necessarily be detected at day 7. An example of a T cell assay timecourse is shown in example 3. For whole proteins, the T cell assay timecourse provides for a sensitive analysis of T cell immunogenicity and thus provides for a sensitive immunogenicity screen for proteins. In addition, as demonstrated in example 3, this assay may also be used to test for the effects of amino acid substitutions on immunogenicity.

The combined approach of using an *in silico* tool for the identification of MHC class II ligands and design of sequence analogues lacking MHC class II ligands, in concert with epitope mapping and re-testing using biologically based assays of T-cell activation is a particularly effective method and most preferred embodiment of the invention. The general method according to this most preferred embodiment comprises the following steps:

i) use of naive T-cell activation assays and synthetic peptides collectively encompassing the protein sequence of interest to identify epitope regions capable of activating T-cells;
ii) use of a computational scheme simulating the binding of the peptide ligand with one or more MHC allotypes to analyse the epitope regions identified in step (i) and thereby identify MHC class II ligands within the epitope region;
iii) use of a computational scheme simulating the binding of the peptide ligand with one or more MHC allotypes to identify sequence analogues of the MHC ligands encompassed within the epitope region(s) which no longer bind MHC class II or bind with lowered affinity to a lesser number of MHC allotypes;
iv) use of naïve T-cell activation assays and synthetic peptides encompassing entirely or in collection encompassing the epitope regions identified within the protein of interest and testing the sequence analogues in naïve T-cell activation assay in parallel with the wild-type (parental) sequences;

It is understood that the software scheme outlined in WO 02/069232 can also be used to define with a high degree of certainty the dataset of all peptides comprising the universe of permissible MHC class ligands for the any human protein such as IFN□. For reasons such as the requirement for proteolytic processing and other physiologic steps leading to the presentation of immunogenic peptides *in vivo,* it would be clear that a relatively minor sub-set of

the entire repertoire of peptides will have ultimate biological relevance. In such situations the inventors have established that *ex vivo* human T-cell activation assays may be used to identify the biologically relevant peptides. Accordingly, synthetic peptides are tested for their ability to evoke a proliferative response in human T-cell cultured *in vitro.* Where this type of approach is conducted using naïve human T-cells taken from healthy donors, the inventors have established that in the operation of such an assay, a stimulation index equal to or greater than 2.0 is a useful measure of induced proliferation. The stimulation index (SI) is conventionally derived by division of the proliferation score (e.g. counts per minute of radioactivity if using for example $^3$H-thymidine incorporation) measured to the test peptide by the score measured in cells not contacted with a test peptide. Peptides which evoke no response give SI= 1.0 although in practice SI values in the range 0.8 - 1.2 are unremarkable. A number of technical proceedures can be inbuilt into the operation of such assays in order to ensure confidence in the recorded scores. Typically all determinations are made at least in triplicate and the mean score may be computed. Where a computed SI =>2.0 individual scores of the triplicate can be examined for evidence of outlying data. Similarly the inclusion of control peptides for which there is expectation that the majority of PBMC donor samples will be responsive may be included in each assay plate. The influenza haemagglutinin peptide 307-309, sequence PKYVKQNTLKLA; and the *Chlamydia* HSP 60 peptide sequence KVVI7QIKKISKPVQH are particularly suitable control peptides although many other examples may be exploited. Assays should preferably also use a potent whole protein antigen such as hemocyanin from Keyhole Limpet to which all PBMC samples would be expected to exhibit an SI significantly greater than 2.0
According to the scheme of the present invention there may be a practical need to test multiple versions of essentially the same peptide sequence in order to establish that the modification, be it a single amino acid substitution or some other change or combination of changes, results in the loss of ability or at least a reduced ability for the peptide(s) to induce a T-cell activation effect. This requirement may be met using a number of different practical approaches one of which could involve the screening of large numbers of variant peptides from the outset and conducting a selection scheme to identify those in which there is a reduced or absent ability to induce proliferation relative to their parental (e.g. wild-type) peptide sequence. Such an approach could be conducted entirely using naive PBMC samples and run concurrently (i.e. in parallel with) the mapping exercise. It is understood that this approach need not be limited to the screening of synthetic peptide species but may be exploited to the screening of whole protein molecules that for example may comprise a multiplicity of variants produced as a "library" of variants from which a desired member is to be selected. Such a library may be produced for example by recombinant means well known in the art or may comprise species produced using synthetic means for example using the principles of combinatorial chemistry. In any event, the desired property to be selected from the library member in this context would be the inability to induce a proliferative response in a PBMC preparation.

Alternatively variant peptides may be screened using naïve PBMC from entirely different donor pool of samples, i.e. epitope mapping is repeated but using modified peptides where there is an expectation for little or no proliferative induction.

A further and particularly favoured scheme would involve the testing of modified peptides for their ability to induce a proliferative effect in an immunological recall assay format. This may be achieved for example using PBMC from a known responding donor identified during the initial naïve PBMC assay phase and stimulating a sample of those cells using a either synthetic peptides (e.g. in a pool) or whole protein followed by a suitable period of culture in the presence of cytokines such as IL-2. Following this incubation, the culture may be re-stimulated using the synthetic (modified) peptide or modified whole protein of interest and the proliferative effect measured using any suitable means. The inventors have classified this assay format as a "recall" assay, so called as the T-cell population responsible for the proliferative response is invoked during a restimulation phase.

The recall type assay is particularly useful in identifying T cell epitopes in protein or peptide antigens that show weak immunogenicity *in vivo* an can provide corroborating evidence for the existence of a T-cell epitope in a given amino acid sequence where the epitope was originally identified by other means, for example by using computational techniques or biological assays. In the operation of such a recall assay, PBMC are isolated from healthy donors or patients with established immune responses to a given therapy. It is necessary to freeze aliquots of autologous PBMC so that they can be used as antigen presenting cells (APC) during subsequent procedures. The assay commences with an antigen priming step. A typical and preferred protocol requires 2-4x$10^6$ PBMC are added to each well of 24 well plate. Either whole protein or peptide antigen or a peptide pool is added to the cells at typical concentrations of 1-10μg/ml and 1-10μM, respectively (total concentration of peptides in peptide pool would be 1μM). The final culture volume is 2ml. The cells are incubated for 7 days where on day 7 10U/ml IL-2 is added and the cells are incubated for a further 3 days whereupon the cells are ready for the antigen re-challenge phase.

The antigen re-challenge requires autologous PBMC as APC. The APC are incubated with whole protein or synthetic peptide antigen (for example at a concentration of 1-10μgl/ml) for 1 hour at 37°C. The proliferative capability of the APC is destroyed most preferably using gamma radiation, for example 4000 rads in a round bottom 96 well plate (1x$10^5$ PBMC/well). 1-10x$10^4$ primed T-cells are added to each well containing the APC's. It is important to set up untreated control reactions comprising antigen primed T-cells cultured with gamma irradiated APC in the absence of

re-challenge antigen. The cells are incubated for 4 days before pulsing proliferation assessment for example 3H-thymidine incorporation assay. It is understood that such a protocol can equally be conducted using enriched or purified populations of cells.

In a third embodiment, there is provided a method whereby a protein antigen may be screened for the presence of determinants within its sequence capable of evoking a T-cell immune response in individuals for whom the protein of interest is to be administered for therapeutic effect against a genetic (constitutional) disease and where, in effect, the protein antigen due to the nature of the genetic deficit in the individuals will constitute a foreign protein. In this sense, the protein is most likely to represent a potent antigen *in vivo* and the inventors have established that it is now readily possible to establish polyclonal or mononclonal T-cell lines in *vitro* from the PBMC of such individuals and these lines may be used as effective reagents in the mapping of T-cell epitopes within proteins. This is achieved in essentially the same way as the recall assay of the foregoing, with the exception that the T cells are subjected to several rounds of antigen stimulation *in vitro* followed immediately by expansion in the presence of IL-2. For establishing polyclonal T cell lines 2-3 rounds of antigen stimulation are generally sufficient to generate a large number of antigen specific cells. These are used to screen large numbers of synthetic peptides (for example in the form of peptide pools), and they may be cryogenically stored to be used at a later date. After the initial round of antigen stimulation comprising co-incubation of the antigen and PBMC for 7 days subsequent re-challenges with antigen are performed in the presence of most preferably autologous irradiated PBMC as antigen presenting cells. These rounds of antigen selection are performed for 3-4 days and are interspersed by expansion phases comprising stimulation with IL-2 which may be added every 3 days for a total period of around 9 days. The final re-challenge is performed using T-cells that have been "rested", that is T cells which have not been IL-2 stimulated for around 4 days. These cells are stimulated with antigen (e.g. synthetic peptide or whole protein) using most preferably autologous antigen presenting cells as previously for around 4 days and the subsequent proliferative response (if any) is measured thereafter.

Accordingly the method of the third embodiment comprises the production of T-cell lines or oligoclonal cultures derived from PBMC samples taken from an individual afflicted with the disease of interest, stimulating *in vitro* said lines or cultures with preparations of synthetic peptides or whole proteins and measuring *in vitro* the proliferative effect if any of individual synthetic peptides or proteins, producing modified variants of individual synthetic peptides or whole proteins and re-testing said modified peptides or proteins for a continued ability to promote a significant proliferative response in the T-cell lines or cultures.

It is particularly useful to establish T-cell lines of oligoclonal cultures from individuals who carry the genetic defect and in whom therapeutic replacement therapy has been initiated to and in whom the replacement therapy has resulted in the induction of an immune response to the therapeutic protein. A prominent example of this kind of subject is provided by individuals undergoing treatment for hemophillia A but in whom there is a significant titre of inhibitory antibodies measurable to the therapeutic Factor VIII. Under the scheme of the present invention it would be particularly desired to exploit PBMC samples from this class of so called "inhibitor patients" as it could be expected that the epitope map of the the Factor VIII protein defined by the T-cell repertoire of a significant number of these individuals will be representative of the most prevalent peptide epitopes that are capable of presentation in the *in vivo* context. In this sense, PBMC from patients in whom there is a previously demonstrated immune response constitute the products of an *in vivo* priming step and are particularly valuable under the scheme of the present. EXAMPLE 4 herein provides detailed description of an epitope mapping programme conducted on human FVIII exploiting both naive human T-cells from healthy donors and PBMCs derived from haemophilia A patients.

Given that the use of PBMC cell lines from individuals previously in receipt of the immunologically foreign protein is in principle a recall assay, it further provides the practical benefit of there being the capacity for a much larger magnitude of proliferative response to any given stimulating peptide or protein. This reduces the technical challenge of conducting a proliferation measurement and in such a situation may give the opportunity for definition of a possible hierarchy of immunodominant epitopes where multiple epitopes are uncovered to a target protein. This is certain to be the case with particularly large proteins such as Factor VIII although as demonstrated herein, small human protein molecules (e.g. less than 200 amino acid residues) may be expected to harbour multiple or complex (i.e. overlapping) T-cell epitopes.

In a fourth embodiment of the present there is provided a scheme whereby the assay format of the foregoing is applied to the screening of production batches of therapeutic biological proteins. The objective of such a screening process is to confirm the consistency of the immunogenic profile of the test biologic and for example may be particularly valuable in situations where the production process for the biologic has been altered by some parameter and although the measured physical properties of the protein may be within accepted ranges, there is a consideration that the potential immunogenic properties of the protein may have been altered. Thus in order to anticipate the generation of an immunogenic response to any new preparation of the molecule of interest the methods set-out herein are particularly effective in providing such a screening procedure.

Under the fourth embodiment therefore, T-cell lines (oligoclonal or mono-clonal) derived as part of the epitope mapping process for the protein of interest or optionally and in addition, a panel of naive PBMC preparations for which

there has been established a population of known responsive preparations, may be used to test the subject protein for immunogenicity *in vitro,* and the responses scored to the test protein are compared to a reference or "gold-standard" preparation of the protein. In this regard where T-cell lines are employed, it is particularly preferred to use lines derived from subjects in whom there has been a demonstrated previous immune response to the reference protein. Such lines are expected to provide a high stimulation index score on antigen challenge *in vitro* and are likely to be representative of the most biologically relevant and immunodominant epitopes within the protein. These lines under the fourth embodiment provide indicators for epitope loss/alteration. By contrast, under the fourth embodiment, panels of naive PBMC containing a known set of responding allotypes to the target protein provide indication of *de novo* epitope generation appearing in the test product protein and are equally valuable in predicting an unwanted clinical immunogenic response.

The term "T-cell epitope" means according to the understanding of this invention an amino acid sequence which is able to bind MHC class II, able to stimulate T-cells and / or also to bind (without necessarily measurably activating) T-cells in complex with MHC class II.

The term "peptide" as used herein and in the appended claims, is a compound that includes two or more amino acids. The amino acids are linked together by a peptide bond (defined herein below). There are 20 different naturally occurring amino acids involved in the biological production of peptides, and any number of them may be linked in any order to form a peptide chain or ring. The naturally occurring amino acids employed in the biological production of peptides all have the L-configuration. Synthetic peptides can be prepared employing conventional synthetic methods, utilizing L-amino acids, D-amino acids, or various combinations of amino acids of the two different configurations. Some peptides contain only a few amino acid units. Short peptides, e.g., having less than ten amino acid units, are sometimes referred to as "oligopeptides". Other peptides contain a large number of amino acid residues, e.g. up to 100 or more, and are referred to as "polypeptides". By convention, a "polypeptide" may be considered as any peptide chain containing three or more amino acids, whereas a "oligopeptide" is usually considered as a particular type of "short" polypeptide. Thus, as used herein, it is understood that any reference to a "polypeptide" also includes an oligopeptide. Further, any reference to a "peptide" includes polypeptides, oligopeptides, and proteins. Each different arrangement of amino acids forms different polypeptides or proteins. The number of polypeptides-and hence the number of different proteins-that can be formed is practically unlimited.

The invention will now be illustrated by the following examples. The examples and foregoing text refer to the following figures:

**Figure 1** shows the immunogenic regions within IFNβ and details the peptide sequences from these regions able to stimulate naive human T-cells.

**Figure 2** provides a table indicating the IFNβ peptides capable of promoting proliferation of naive human T-cells *in vitro.* For two of the donors, responses are recorded to multiple overlapping peptides from either region R1 or R2. Responses to individual synthetic peptides mapping to epitope regions R1 or R2 are scored from six donors.

**Figure 3** provides exemplary data from time course T-cell activation assays. Charts plot stimulation index (SI) against time (days) for synthetic peptides derived from the IFNα R1, R2 and R3 epitope regions and analogue peptide sequences containing amino acid substitutions tested in parallel.

**EXAMPLE 1**

The interaction between MHC, peptide and T-cell receptor (TCR) provides the structural basis for the antigen specificity of T-cell recognition. T-cell proliferation assays test the binding of peptides to MHC and the recognition of MHC/peptide complexes by the TCR. *In vitro* T-cell proliferation assays of the present example, involve the stimulation of peripheral blood mononuclear cells (PBMCs), containing antigen presenting cells (APCs) and T-cells. Stimulation is conducted *in vitro* using synthetic peptide antigens, and in some experiments whole protein antigen. Stimulated T-cell proliferation is measured using $^{3}$H-thymidine ($^{3}$H-Thy) and the presence of incorporated $^{3}$H-Thy assessed using scintillation counting of washed fixed cells.

Buffy coats from human blood stored for less than 12 hours were obtained from the National Blood Service (Addenbrooks Hospital, Cambridge, UK). Ficoll-paque was obtained from Amersham Pharmacia Biotech (Amersham, UK). Serum free AIM V media for the culture of primary human lymphocytes and containing L-glutamine, 50μg/ml streptomycin, 10μg/ml gentomycin and 0.1% human serum albumin was from Gibco-BRL (Paisley, UK). Synthetic peptides were obtained from Pepscan (The Netherlands) and Babraham Technix (Cambridge, UK).

Erythrocytes and leukocytes were separated from plasma and platelets by gentle centrifugation of buffy coats. The top phase (containing plasma and platelets) was removed and discarded. Erythrocytes and leukocytes were diluted 1:1 in phosphate buffered saline (PBS) before layering onto 15ml ficoll-paque (Amersham Pharmacia, Amersham UK). Centrifugation was done according to the manufacturers recommended conditions and PBMCs were harvested from

the serum+PBS/ficoll paque interface. PBMCs were mixed with PBS (1:1) and collected by centrifugation. The supernatant was removed and discarded and the PBMC pellet resuspended in 50ml PBS. Cells were again pelleted by centrifugation and the PBS supernatant discarded. Cells were resuspended using 50ml AIM V media and at this point counted and viability assessed using trypan blue dye exclusion. Cells were again collected by centrifugation and the supernatant discarded. Cells were resuspended for cryogenic storage at a density of $3x10^7$ per ml.
The storage medium was 90%(v/v) heat inactivated AB human serum (Sigma, Poole, UK) and 10%(v/v) DMSO (Sigma, Poole, UK). Cells were transferred to a regulated freezing container (Sigma) and placed at -70°C overnight before transferring to liquid $N_2$ for long term storage. When required for use, cells were thawed rapidly in a water bath at 37°C before transferring to 10ml pre-warmed AIM V medium.

PBMC were stimulated with protein and peptide antigens in a 96 well flat bottom plate at a density of $2x10^5$ PBMC per well. PBMC were incubated for 7 days at 37°C before pulsing with $^3$H-Thy (Amersham-Phamacia, Amersham, UK). For the present study, synthetic peptides (15mers) that overlapped by 3aa increments were generated that spanned the entire sequence of IFNβ. Peptide identification numbers (ID#) and sequences are given in Table 1.

**Table 1 IFNβ peptides**

| Peptide ID Number | IFNβ-1a; 15mer IFNβ-1a; 15mer sequence | Peptide ID Number | IFNβ-1a; 15mer IFNβ-1a; 15mer sequence |
|---|---|---|---|
| 1 | MSYNLLGFLQRSSNF | 28 | TIVENLLANVYHQIN |
| 2 | NLLGFLQRSSNFQCQ | 29 | ENLLANVYHQINHLK |
| 3 | GFLQRSSNFQCQKLL | 30 | LANVYHQINHLKTVL |
| 4 | QRSSNFQCQKLLWQL | 31 | VYHQINHLKTVLEEK |
| 5 | SNFQCQKLLWQLNGR | 32 | QINHLKTVLEEKLEK |
| 6 | QCQKLLWQLNGRLEY | 33 | HLKTVLEEKLEKEDF |
| 7 | KLLWQLNGRLEYCLK | 34 | TVLEEKLEKEDFTRG |
| 8 | WQLNGRLEYCLKDRM | 35 | EEKLEKEDFTRGKLM |
| 9 | NGRLEYCLKDRMNFD | 36 | LEKEDFTRGKLMSSL |
| 10 | LEYCLKDRMNFDIPE | 37 | EDFTRGKLMSSLHLK |
| 11 | CLKDRMNFDIPEEIK | 38 | TRGKLMSSLHLKRYY |
| 12 | DRMNFDIPEEIKQLQ | 39 | KLMSSLHLKRYYGRI |
| 13 | NFDIPEEIKQLQQFQ | 40 | SSLHLKRYYGRILHY |
| 14 | IPEEIKQLQQFQKED | 41 | HLKRYYGRILHYLKA |
| 15 | EIKQLQQFQKEDAAL | 42 | RYYGRILHYLKAKEY |
| 16 | QLQQFQKEDAALTIY | 43 | GRILHYLKAKEYSHC |
| 17 | QFQKEDAALTIYEML | 44 | LHYLKAKEYSHCAWT |
| 18 | KEDAALTIYEMLQNI | 45 | LKAKEYSHCANTIVR |
| 19 | AALTIYEMLQNIFAI | 46 | KEYSHCAWTIVRVEI |
| 20 | TIYEMLQNIFAIFRQ | 47 | SHCAWTIVRVEILRN |
| 21 | EMLQNIFAIFRQDSS | 48 | AWTIVRVEILRNFYF |
| 22 | QNIFAIFRQDSSSTG | 49 | IVRVEILRNFYFINR |
| 23 | FAIFRQDSSSTGWNE | 50 | VEILRNFYFINRLTG |
| 24 | FRQDSSSTGWNETIV | 51 | LRNFYFINRLTGYLR |
| 25 | DSSSTGWNETIVENL | | |
| 26 | STGWNETIVENLLAN | | |
| 27 | WNETIVENLLANVYH | | |

Each peptide was screened individually against PBMC's isolated from 20 naïve donors. Two control peptides that have previously been shown to be immunogenic and a potent non-recall antigen KLH were used in each donor assay. The control antigens used in this study were Flu haemagglutinin 307-319 (sequence: PKYVKQNTLKLAT); Chlamydia HSP 60 peptide (sequence: KWDQIKKISKPVQH) and Keyhole Limpet hemocyanin.

Peptides were dissolved in DMSO to a final concentration of 10mM, these stock solutions were then diluted 1/500 in AIM V media (final concentration 20μM). Peptides were added to a flat bottom 96 well plate to give a final concentration of 2 and 20μM in a 100μl. The viability of thawed PBMC's was assessed by trypan blue dye exclusion, cells were then resuspended at a density of $2x10^6$ cells/ml, and 100μl ($2x10^5$ PBMC/well) was transferred to each well containing peptides. Triplicate well cultures were assayed at each peptide concentration. Plates were incubated for 7

days in a humidified atmosphere of 5% $CO_2$ at 37°C. Cells were pulsed for 18-21 hours with 1μCi $^3$H-Thy/well before harvesting onto filter mats. CPM values were determined using a Wallac microplate beta top plate counter (Perkin Elmer). Results were expressed as stimulation indices, where the stimulation index (SI) is derived by division of the proliferation score (e.g. counts per minute of radioactivity) measured to the test peptide by the score measured in cells not contacted with a test peptide.

Mapping T cell epitopes in the IFNβ sequence using the T cell proliferation assay resulted in the identification of two immunogenic regions R1 and R2 resulting, in each case, by responses to four overlapping peptides (Figure 1).

**EXAMPLE 2**

An epitope map for the human protein interferon a2 (IFNα) was derived using the method of EXAMPLE 1. In all respects the method was as per EXAMPLE 1 except that synthetic peptides were as given in Table 2 (below) and incubation with the PBMC preparations was at a concentration of 10uM

Mapping T cell epitopes in the IFNα sequence resulted in the identification of three immunogenic regions R1, R2, R3. This was determined by T cell proliferation to seven, four and five overlapping peptides respectively as shown in Figure 2. Region 3 is considered to contain a potential immunodominant T-cell epitope as proliferation is scored in two thirds of donors that responded to IFNα peptides.

**Table 2: IFNα peptides**

| Peptide ID Number | IFNα2b; 15mer sequence | | Peptide ID Number | IFNα2b; 15mer sequence |
|---|---|---|---|---|
| 1 | CDLPQTHSLGSRRTL | | 28 | DKFYTELYQQLNDLE |
| 2 | PQTHSLGSRRTLMLL | | 29 | YTELYQQLNDLEACV |
| 3 | HSLGSRRTLMLLAQM | | 30 | LYQQLNDLEACVIQG |
| 4 | GSRRTLMLLAQMRRI | | 31 | QLNDLEACVIQGVGV |
| 5 | RTLMLLAQMRRISLF | | 32 | DLEACVIQGVGVTET |
| 6 | MGLAQMRRISLFSCL | | 33 | ACVIQGVGVTETPLM |
| 7 | AQMRRISLFSCLKDR | | 34 | IQGVGVTETPLMKJED |
| 8 | RRISLFSCLKDRHDF | | 35 | VGVTETPLMKEDSIL |
| 9 | SLFSCLKDRHDFGFP | | 36 | TETPLMKEDSILAVR |
| 10 | SCLKDRHDFGFPQEE | | 37 | PLMKEDSILAVRKYF |
| 11 | KDRHDFGFPQEEFGN | | 38 | REDSILAVRKYFQRI |
| 12 | HDFGFPQEEFGNQFQ | | 39 | SILAVRKYFQRITLY |
| 13 | GFPQEEFGNQFQKAE | | 40 | AVRKYFQRITLYLKE |
| 14 | QEEFGNQFQKAETIP | | 41 | KYFQRITLYLKEKKY |
| 15 | FGNQFQKAETIPVLH | | 42. | QRITLYLKEKKYSPC |
| 16 | QFQKAETIPVLHEMI | | 43 | TLYLKEKKYSPCAWE |
| 17 | KAETIPVLHEMIQQI | | 44 | LKEKKYSPCAWEVVR |
| 18 | TIPVLHEMIQQIFNL | | 45 | KKYSPCAWEWRAEI |
| 19 | VLHEMIQQIFNLFST | | 46 | SPCAWEVVRAEIMRS |
| 20 | EMIQQIFNLFSTKDS | | 47 | AWEVVRAEIMRSFSL |
| 21 | QQIFNLFSTKDSSAA | | 48 | VVRAEIMRSFSLSTN |
| 22 | FNLFSTKDSSAAWDE | | 49 | AEIMRSFSLSTNLQE |
| 23 | FSTKDSSAAWDETLL | | 50 | MRSFSLSTNLQESLR |
| 24 | KDSSAAWDETLLDKF | | 51 | FSLSTNLQESLRSKE |
| 25 | SAAWDETLLDKFYTE | | | |

Table continued

| Peptide ID Number | IFNα2b; 15mer sequence | | Peptide ID Number | IFNα2b; 15mer sequence |
|---|---|---|---|---|
| 26 | WDETLLDKFYTELYQ | | | |
| 27 | TLLDKFYTELYQQLN | | | |

**EXAMPLE 3**

*Method for conducting a time course T-cell activation assay*

A general protocol for conducting a time course T-cell activation assay comprises the following steps:

1. Thaw 1 vial of PBMC per donor
2. Resuspend cells at 2-4x$10^6$ cells/ml (in AIM V).
3. Transfer 1ml to 3 wells of a 24 well plate (giving a final concentration of 2-4x$10^6$ PBMC/well), since it is usual to test the antigen at two different concentrations and compare against a non-antigen treated control (e.g. 10-50ug/ml protein or 1-5uM peptide).
4. Make stock solutions of antigens typically 100ug/ml for proteins and 2-10uM for peptides. Add 1ml of antigen to each well to give a final concentration 10-50ug/ml protein or 1-5uM peptide.
5. Incubate for 5 days.
6. Gently resuspend the cells in the 2ml cultures by pipetting and from each condition remove 100ul cells and place into a well of 96 well plate (round bottom), repeat this three time of reach culture condition (total of 300ul removed from each culture condition per time point).
7. To each well of cells in the 96 well plate add 1μCi/well 3H[Thy] in 100ul AIM V.
8. Incubate overnight and harvest.
9. Repeat stage 6-8 for days 6, 7, and 8 (day 9 can be included if necessary).
10. Make SI determinations and plot the SI versus time for each antigen.

**FIGURE** 3 shows typical results for the timecourse assay for immunogenicity of long peptides spanning the immunogenic regions of interferon α2 (cf Example 2). This novel timecourse method is especially useful for analysis of whole proteins as a screen for T cell immunogenicity (SI's >1.8) and to analyse the effects on immunogenicity of amino acid modifications within the protein.

**EXAMPLE 4**

*Method for establishment of T cell lines and clones.*

Peripheral blood mononuclear cells (PBMC) were isolated from blood obtained from haemophiliac patients, and cryogenically stored under liquid nitrogen.
Blood samples were provided with fully informed consent and working under local ethical approval of the Addenbrooke's Health Care Trust.

T cell lines were established by stimulating antigen specific T cells in bulk cultures using FVIII followed by several cycles of IL-2 induced expansion. Initially PBMC were incubated (at 37°C in a humidified atmosphere of 5% $CO_2$) at 2x$10^6$ in 2ml AIM V media containing 4ug/ml FVIII (Refacto™) in 24 well plates. After 7 days incubation 100U/ml IL-2 was added and cultures were incubated for further 3 days. T blasts were collected and counted upon completion of the 10 day antigen/IL-2 stimulation. In order to retain antigen specificity T blasts were subjected to a second round of antigen stimulation using γ-irradiated autologous PBMC as antigen presenting cells. This was achieved by incubating 1x$10^6$ autologous PBMC/well in a 24 well plate with 4μg/ml FVIII for 1 hour in 0.75ml AIM V (containing 5% heat inactivated human AB serum) before being subjected to 4000 rads γ-irradiation. Autologous T blasts were added in 0.25ml AIM V at 4x$10^5$ cells/ml to the γ-irradiated antigen presenting cells (pre-loaded with FVIII) and incubated for 3 days. T blasts were expanded by stimulating cells with 100U/ml IL-2 for 3 days; cultures were then supplied with fresh IL-2 (final concentration of 100U/ml) at 3 day intervals for a total of 9 days. To ensure that all expanded T blasts were antigen specific a third round of antigen stimulation was performed, where T blasts were collected and resuspended at 4x$10^5$cells/ml in AIM V media. As described before antigen presenting cells were generated by incubating 1x$10^6$ γ-irradiated autologous PBMC in a 24 well plate with 4ug/ml FVIII for 1 hour in 0.75ml AIM V (containing 5% heat inactivated human AB serum). Autologous T blasts in 0.25ml AIM V at 4x$10^5$ cells/ml were added to the γ-irradiated antigen presenting cells and incubated for 3 days. A final expansion in 10U/ml IL-2 was performed 3 days before T blasts were collected

and used to screen peptide pools.

*Cloning from Bulk Cultures*

After the third stimulation with FVIII antigen T blasts were collected and resuspended by serial dilution to a density of $4x10^2$-$1x10^4$ cells/ml (2 x final culture density). Autologous PBMC were thawed and resuspended to $2x10^6$ cells/ml (2 x final culture density) in a polyproplene tube. PBMC were then exposed to 4000 rads γ-irradiation and were used as antigen presenting cells to select antigen reactive T cell clones by limiting dilution. γ-irradiated antigen presenting cells ($1x10^6$ final density) were mixed with the T blasts ($2x10^2$-$5x10^3$ final density), 1-10μg/ml FVIII antigen and 100U/ml IL-2. T cell clones were established in Terasaki plates by adding 20μl of the APC, T blast, FVIII and IL-2 mixture to each well. Limiting dilution cloning was performed using 2-50 T blasts/well of a Terasaki plate.

*Selection and Maintenance of T Cell Clones*

T blasts were incubated with FVIII antigen, IL-2 and γ-irradiated autologous antigen presenting cells for approximately 14 days. After identifying wells that contained cells showing unequivocal growth, T blasts were transferred to a single well of a round bottom 96 well plate containing $1x10^5$ γ-irradiated allogenic PBMC, 100U/ml IL-2 and 1μg/ml phytohaemaglutinin (PHA) in a final volume 200μl AIM V (with 1% heat inactivated human AB serum). T cell clones were split when cells became confluent, and ultimately transferred to a single well of 24 well plate containing$1x10^6$ γ-irradiated allogenic PBMC (feeder cells), 100U/ml IL-2 and 1μg/ml phytohaemaglutinin (PHA) in a final volume of 2ml AIM V (with 1% heat inactivated human AB serum). Routine maintenance of T cell clones involved stimulation with fresh PHA and allogenic feeder cells every 2-3 weeks (depending on cell growth) and twice weekly stimulation with 10OU/ml IL-2. Only T cell clones that proved to be FVIII specific were expanded and used to screen FVIII peptides.

*EBV Transformation of Autologous B Cells.*

B cells from PBMC preparations were immortalized to generate B lymphoblastoid cell lines (BLCL) by adding 3ml of filtered (0.45μ) B95.8 supernatant to $4x10^6$ PBMC and incubating at 37°C for 1 hour. PBMC were pelleted and resuspended in 2ml RPMI containing 5% heat-inactive foetal calf serum (PCS) and 1μg/ml cyclosporin A. After 7 days incubation 1ml of culture media was replaced with fresh RPMI containing 5% FCS and 2μg/ml cyclosporin A (to give a final concentration of 1μg/ml cyclosporin A). This feeding regime was repeated on days 14 and 21 after which cells were split when necessary using RPMI. containing 5% FCS and expanded into tissue culture flasks.

*Screening FVIII Peptides Using T Cell Lines/Clones*

Peptides of 15 residues in length and overlapping with the previous peptide by increments of 12 amino acids were synthesized (Pepscan, Netherlands). Peptides were initially solubilized at 10mM in 100% dimethylsulphoxide (DMSO) for storage. Peptide pools were generated to simultaneously screen a large number of peptides against FVIII specific T cell lines. Pools were organized such that each pool contained overlapping peptides of subsequent pools by using this approach T cell epitopes that overlap two peptides will result in inducing proliferation two separate pools. Each pool typically consisted of 8 peptides with each.peptide being tested at either 1 or 5μM.

Autologous PBMC (for T cell lines) or EBV transformed BLCL (for T cell clones) were used as antigen presenting cells by re-suspending $1x10^5$ PBMC or BLCL in 50μl AIM V media which was then added to each well of a round bottom 96 well plate. Peptide pools were added in triplicate wells for each pool at both concentrations (1 or 5μM). Antigen presenting cells and peptide pools were incubated for 1 hour at 37°C before exposure to 4000 rads γ-irradiation. BLCL were pre-treated with 1μg/ml Mitomycin C for 1 hour at 37°C followed by washing 4 times in AIM V when used as antigen presenting cells (instead of γ-irradiated autologous PBMC) for T cell clones. Antigen specific T cell lines or T cell clones were then added at $5x10^4$ cells per well and the cultures were incubated for 3 days. On the third day each well was pulsed with 1μCi [$^3$H]-Thymidine for a minimum of 8 hours. After harvesting the plates onto filtermats the cpm/well was determined using a Wallac Microplate Beta counter.

*Naïve T Cell Epitope Map using PBMC from Healthy Donors*

Blood from 40 healthy HLA-DR typed donors was used to isolate PBMC which were used to screen individual FVIII peptides at two concentrations (1 and 5μM). Since there were insufficient numbers of PBMC from each donor to screen all FVIII peptides, donors were split into two groups where the first 20 donors were used to screen peptides spanning the first half of the molecule and the second set of donors used to screen the remaining peptides. Donors were selected according to MHC class II allotypes expressed in order to cover a large number of allotypes present in the world

population. MHC allotypes were detected using

The tissue types for all PBMC samples were assayed using a commercially available reagent system (Dynal, Wirral, UK). Assays were conducted in accordance with the suppliers recommended protocols and standard ancillary reagents and agarose electrophoresis systems.

PBMC contain physiological numbers of naïve T cells and antigen presenting cells. These cells were used at a density of 2x$10^5$ cells/well (96 flat bottom plate) to screen peptides at 1 and 5 μM in triplicate 200μl cultures. Cells were incubated with peptides at 37°C for 6 days before pulsing each well with 1μCi [$^3$H]-Thymidine for a minimum of 8 hours. Cultures were harvested onto filtermats and the cpm/well was determined using a Wallac Microplate Beta counter.

TABLE 3 shows an epitope map for human B-domain deleted FVIII generated using T cell lines from haemophiliacs and naive T-cell preparations from healthy individuals. Where T blasts and naïve PBMC derived T-cells were used to identify peptide pools containing T cell epitopes, those pools were then decoded to identify the individual peptide containing the T cell epitope.

**TABLE 3**

| Residue #[+] | Peptide Sequence |
|---|---|
| 196 | ILLFAVFDEGKSWSH |
| 406 | SYKSQYLNNGPQRIG |
| 415 | GPQRIGRtCYKKVRFM |
| 511 | YKWTVTVRDGPTKSD |
| 610 | ASNIMHSINGYVFDS |
| 634 | VAYWYILSIGAQTDF |
| 817 | MSSSPHVLRNRAQSG |
| 1009 | CNIQMEDPTFKENYR |
| 1127 | STLFLVYSNKCQTPL |
| 1204 | ISQFIIMYSLDGKKW |
| 1251 | IARYIRLHPTHYSIRSTLRM |
| • Sequence numbering according to B domain deleted sequence | |

**EXAMPLE 5:**

*Computational scheme*

There are a number of factors that play important roles in determining the total structure of a protein or polypeptide. First, the peptide bond, i.e., that bond which joins the amino acids in the chain together, is a covalent bond. This bond is planar in structure, essentially a substituted amide. An "amide" is any of a group of organic compounds containing the grouping -CONH-.

The planar peptide bond linking Cα of adjacent amino acids may be represented as

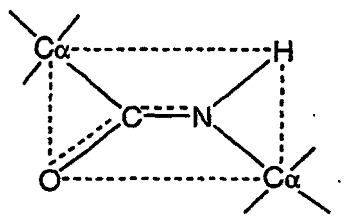

depicted below:

Because the O=C and the C-N atoms lie in a relatively rigid plane, free rotation does not occur about these axes. Hence, a plane schematically depicted by the interrupted line is sometimes referred to as an "amide" or "peptide

plane" plane wherein lie the oxygen (O), carbon (C), nitrogen (N), and hydrogen (H) atoms of the peptide backbone. At opposite corners of this amide plane are located the Cα atoms. Since there is substantially no rotation about the O=C and C-N atoms in the peptide or amide plane, a polypeptide chain thus comprises a series of planar peptide linkages joining the Cα atoms.

A second factor that plays an important role in defining the total structure or conformation of a polypeptide or protein is the angle of rotation of each amide plane about the common Cα linkage. The terms "angle of rotation" and "torsion angle" are hereinafter regarded as equivalent terms. Assuming that the O, C, N, and H atoms remain in the amide plane (which is usually a valid assumption, although there may be some slight deviations from planarity of these atoms for some conformations), these angles of rotation define the N and R polypeptide's backbone conformation, i.e., the structure as it exists between adjacent residues. These two angles are known as $\phi$ and $\psi$. A set of the angles $\phi 1$, $\psi_1$, where the subscript i represents a particular residue of a polypeptide chain, thus effectively defines the polypeptide secondary structure. The conventions used in defining the $\phi$, $\psi$ angles, i.e., the reference points at which the amide planes form a zero degree angle, and the definition of which angle is $\phi$, and which angle is $\psi$, for a given polypeptide, are defined in the literature. See, e.g,, Ramachandran et al. *Adv. Prot. Chem.* 23:283-437 (1968), at pages 285-94, which pages are incorporated herein by reference. The present method can be applied to any protein, and is based in part upon the discovery that in humans the primary Pocket 1 anchor position of MHC Class II molecule binding grooves has a well designed specificity for particular amino acid side chains. The specificity of this pocket is determined by the identity of the amino acid at position 86 of the beta chain of the MHC Class II molecule. This site is located at the bottom of Pocket 1 and determines the size of the side chain that can be accommodated by this pocket. Marshall, K.W., *J. Immunol.,* 152:4946-4956 (1994). If this residue is a glycine, then all hydrophobic aliphatic and aromatic amino acids (hydrophobic aliphatics being: valine, leucine, isoleucine, methionine and aromatics being: phenylalanine, tyrosine and tryptophan) can be accommodated in the pocket, a preference being for the aromatic side chains. If this pocket residue is a valine, then the side chain of this amino acid protrudes into the pocket and restricts the size of peptide side chains that can be accommodated such that only hydrophobic aliphatic side chains can be accommodated. Therefore, in an amino acid residue sequence, wherever an amino acid with a hydrophobic aliphatic or aromatic side chain is found, there is the potential for a MHC Class II restricted T-cell epitope to be present. If the side-chain is hydrophobic aliphatic, however, it is approximately twice as likely to be associated with a T-cell epitope than an aromatic side chain (assuming an approximately even distribution of Pocket 1 types throughout the global population).

A computational method embodying the present invention profiles the likelihood of peptide regions to contain T-cell epitopes as follows:

(1) The primary sequence of a peptide segment of predetermined length is scanned, and all hydrophobic aliphatic and aromatic side chains present are identified. (2)The hydrophobic aliphatic side chains are assigned a value greater than that for the aromatic side chains; preferably about twice the value assigned to the aromatic side chains, e.g., a value of 2 for a hydrophobic aliphatic side chain and a value of 1 for an aromatic side chain. (3) The values determined to be present are summed for each overlapping amino acid residue segment (window) of predetermined uniform length within the peptide, and the total value for a particular segment (window) is assigned to a single amino acid residue at an intermediate position of the segment (window), preferably to a residue at about the midpoint of the sampled segment (window). This procedure is repeated for each sampled overlapping amino acid residue segment (window). Thus, each amino acid residue of the peptide is assigned a value that relates to the likelihood of a T-cell epitope being present in that particular segment (window). (4) The values calculated and assigned as described in Step 3, above, can be plotted against the amino acid coordinates of the entire amino acid residue sequence being assessed. (5) All portions of the sequence which have a score of a predetermined value, e.g., a value of 1, are deemed likely to contain a T-cell epitope and can be modified, if desired.

This particular aspect of the present invention provides a general method by which the regions of peptides likely to contain T-cell epitopes can be described. Modifications to the peptide in these regions have the potential to modify the MHC Class II binding characteristics.

According to another aspect of the present invention, T-cell epitopes can be predicted with greater accuracy by the use of a more sophisticated computational method which takes into account the interactions of peptides with models of MHC Class II alleles.

The computational prediction of T-cell epitopes present within a peptide according to this particular aspect contemplates the construction of models of at least 42 MHC Class II alleles based upon the structures of all known MHC Class II molecules and a method for the use of these models in the computational identification of T-cell epitopes, the construction of libraries of peptide backbones for each model in order to allow for the known variability in relative peptide backbone alpha carbon (Cα) positions, the construction of libraries of amino-acid side chain conformations for each backbone dock with each model for each of the 20 amino-acid alternatives at positions critical for the interaction between peptide and MHC Class II molecule, and the use of these libraries of backbones and side-chain conformations in conjunction with a scoring function to select the optimum backbone and side-chain conformation for a particular peptide docked with a particular MHC Class II molecule and the derivation of a binding score from this interaction.

Models of MHC Class II molecules can be derived via homology modeling from a number of similar structures found in the Brookhaven Protein Data Bank ("'PDB"). These may be made by the use of semi-automatic homology modeling software (Modeller, Sali A. & Blundell TL., 1993. *J. Mol Biol* 234:779-815) which incorporates a simulated annealing function, in conjunction with the CHARMm force-field for energy minimisation (available from Molecular Simulations Inc., San Diego, Ca.). Alternative modeling methods can be utilized as well.

The present method differs significantly from other computational methods which use libraries of experimentally derived binding data of each amino-acid alternative at each position in the binding groove for a small set of MHC Class II molecules (Marshall, K.W., *et al., Biomed Pept. Proteins Nucleic Acids,* 1(3):157-162) (1995) or yet other computational methods which use similar experimental binding data in order to define the binding characteristics of particular types of binding pockets within the groove, again using a relatively small subset of MHC Class II molecules, and then 'mixing and matching' pocket types from this pocket library to artificially create further 'virtual' MHC Class II molecules (Sturniolo T., et al., *Nat. Biotech,* 17(6): 555-561 (1999). Both prior methods suffer the major disadvantage that, due to the complexity of the assays and the need to synthesize large numbers of peptide variants, only a small number of MHC Class II molecules can be experimentally scanned. Therefore the first prior method can only make predictions for a small number of MHC Class II molecules. The second prior method also makes the assumption that a pocket lined with similar amino-acids in one molecule will have the same binding characteristics when in the context of a different Class II allele and suffers further disadvantages in that only those MHC Class II molecules can be 'virtually' created which contain pockets contained within the pocket library. Using the modeling approach described herein, the structure of any number and type of MHC Class II molecules can be deduced, therefore alleles can be specifically selected to be representative of the global population. In addition; the number of MHC Class II molecules scanned can be increased by making further models further than having to generate additional data via complex experimentation.

The use of a backbone library allows for variation in the positions of the C$\alpha$ atoms of the various peptides being scanned when docked with particular MHC Class II molecules.

This is again in contrast to the alternative prior computational methods described above which rely on the use of simplified peptide backbones for scanning amino-acid binding in particular pockets. These simplified backbones are not likely to be representative of backbone conformations found in 'real' peptides leading to inaccuracies in prediction of peptide binding. The present backbone library is created by superposing the backbones of all peptides bound to MHC Class II molecules found within the Protein Data Bank and noting the root mean square (RMS) deviation between the C$\alpha$ atoms of each of the eleven amino-acids located within the binding groove. While this library can be derived from a small number of suitable available mouse and human structures (currently 13), in order to allow for the possibility of even greater variability, the RMS figure for each C"-$\alpha$ position is increased by 50%. The average C$\alpha$ position of each amino-acid is then determined and a sphere drawn around this point whose radius equals the RMS deviation at that position plus 50%. This sphere represents all allowed C$\alpha$ positions.

Working from the C$\alpha$ with the least RMS deviation (that of the amino-acid in Pocket 1 as mentioned above, equivalent to Position 2 of the 11 residues in the binding groove), the sphere is three-dimensionally gridded, and each vertex within the grid is then used as a possible location for a C$\alpha$ of that amino-acid. The subsequent amide plane, corresponding to the peptide bond to the subsequent amino-acid is grafted onto each of these C$\alpha$s and the $\phi$ and $\psi$ angles are rotated step-wise at set intervals in order to position the subsequent C$\alpha$. If the subsequent C$\alpha$ falls within the 'sphere of allowed positions' for this C$\alpha$ than the orientation of the dipeptide is accepted, whereas if it falls outside the sphere then the dipeptide is rejected. This process is then repeated for each of the subsequent C$\alpha$ positions, such that the peptide grows from the Pocket 1 C$\alpha$ 'seed', until all nine subsequent C$\alpha$s have been positioned from all possible permutations of the preceding C$\alpha$s. The process is then repeated once more for the single C$\alpha$ preceding pocket 1 to create a library of backbone C$\alpha$ positions located within the binding groove.

The number of backbones generated is dependent upon several factors: The size of the 'spheres of allowed positions'; the fineness of the gridding of the 'primary sphere' at the Pocket 1 position; the fineness of the step-wise rotation of the $\phi$ and $\psi$ angles used to position subsequent C$\alpha$s. Using this process, a large library of backbones can be created. The larger the backbone library, the more likely it will be that the optimum fit will be found for a particular peptide within the binding groove of an MHC Class II molecule. Inasmuch as all backbones will not be suitable for docking with all the models of MHC Class II molecules due to clashes with amino-acids of the binding domains, for each allele a subset of the library is created comprising backbones which can be accommodated by that allele. The use of the backbone library, in conjunction with the models of MHC Class II molecules creates an exhaustive database consisting of allowed side chain conformations for each amino-acid in each position of the binding groove for each MHC Class II molecule docked with each allowed backbone. This data set is generated using a simple steric overlap function where a MHC Class II molecule is docked with a backbone and an amino-acid side chain is grafted onto the backbone at the desired position. Each of the rotatable bonds of the side chain is rotated step-wise at set intervals and the resultant positions of the atoms dependent upon that bond noted. The interaction of the atom with atoms of side-chains of the binding groove is noted and positions are either accepted or rejected according to the following criteria: The sum total of the overlap of all atoms so far positioned must not exceed a pre-determined value. Thus the stringency of the conformational search is a function of the interval used in the step-wise rotation of the bond and the pre-determined limit for the total overlap. This latter value can be small if it is known that a particular pocket is rigid, however the stringency can be relaxed if the positions of pocket side-chains are known to be relatively flexible. Thus allowances can be made to imitate variations in flexibility within pockets of the binding groove. This conformational search is then repeated for every amino-acid at every position of each backbone when docked with each of the MHC Class II molecules to create the exhaustive database of side-chain conformations.

A suitable mathematical expression is used to estimate the energy of binding between models of MHC Class II molecules in conjunction with peptide ligand conformations which have to be empirically derived by scanning the large database of backbone/side-chain conformations described above. Thus a protein is scanned for potential T-cell epitopes by subjecting each possible peptide of length varying between 9 and 20 amino-acids (although the length is kept constant for each scan) to the following computations: An MHC Class II molecule is selected together with a peptide backbone allowed for that molecule and the side-chains corresponding to the desired peptide sequence are grafted on. Atom identity and interatomic distance data relating to a particular side-chain at a particular position on the backbone are collected for each allowed conformation of that amino-acid (obtained from the database described above). This is repeated for each side-chain along the backbone and peptide scores derived using a scoring function. The best score for that backbone is retained and the process repeated for each allowed backbone for the selected model. The scores from all allowed backbones are compared and the highest score is deemed to be the peptide score for the desired peptide in that MHC Class II model. This process is then repeated for each model with every possible peptide derived from the protein being scanned, and the scores for peptides versus models are displayed.

In the context of the present invention, each ligand presented for the binding affinity calculation is an amino-acid segment selected from a peptide or protein as discussed above. Thus, the ligand is a selected stretch of amino acids about 9 to 20 amino acids in length derived from a peptide, polypeptide or protein of known sequence. The terms "amino acids" and "residues" are hereinafter regarded as equivalent terms. The ligand, in the form of the consecutive amino acids of the peptide to be examined grafted onto a backbone from the backbone library, is positioned in the binding cleft of an MHC Class II molecule from the MHC Class II molecule model library via the coordinates of the C"-α atoms of the peptide backbone and an allowed conformation for each side-chain is selected from the database of allowed conformations. The relevant atom identities and interatomic distances are also retrieved from this database and used to calculate the peptide binding score. Ligands with a high binding affinity for the MHC Class II binding pocket are flagged as candidates for site-directed mutagenesis. Amino-acid substitutions are made in the flagged ligand (and hence in the protein of interest) which is then retested using the scoring function in order to determine changes which reduce the binding affinity below a predetermined threshold value. These changes can then be incorporated into the protein of interest to remove T-cell epitopes.

Binding between the peptide ligand and the binding groove of MHC Class II molecules involves non-covalent interactions including, but not limited to: hydrogen bonds, electrostatic interactions, hydrophobic (lipophilic) interactions and Van der Walls interactions. These are included in the peptide scoring function as described in detail below. It should be understood that a hydrogen bond is a non-covalent bond which can be formed between polar or charged groups and consists of a hydrogen atom shared by two other atoms. The hydrogen of the hydrogen donor has a positive charge where the hydrogen acceptor has a partial negative charge. For the purposes of peptide/protein interactions, hydrogen bond donors may be either nitrogens with hydrogen attached or hydrogens attached to oxygen or nitrogen. Hydrogen bond acceptor atoms may be oxygens not attached to hydrogen, nitrogens with no hydrogens attached and one or two connections, or sulphurs with only one connection. Certain atoms, such as oxygens attached to hydrogens or imine nitrogens (e.g. C=NH) may be both hydrogen acceptors or donors. Hydrogen bond energies range from 3 to 7 Kcal/mol and are much stronger than Van der Waal's bonds, but weaker than covalent bonds. Hydrogen bonds are also highly directional and are at their strongest when the donor atom, hydrogen atom and acceptor atom are co-linear. Electrostatic bonds are formed between oppositely charged ion pairs and the strength of the interaction is inversely proportional to the square of the distance between the atoms according to Coulomb's law. The optimal distance between ion pairs is about 2.8Å. In protein/peptide interactions, electrostatic bonds may be formed between arginine, histidine or lysine and aspartate or glutamate. The strength of the bond will depend upon the pKa of the ionizing group and the dielectric constant of the medium although they are approximately similar in strength to hydrogen bonds.

Lipophilic interactions are favorable hydrophobic-hydrophobic contacts that occur between he protein and peptide ligand. Usually, these will occur between hydrophobic amino acid side chains of the peptide buried within the pockets of the binding groove such that they are not exposed to solvent. Exposure of the hydrophobic residues to solvent is highly unfavorable since the surrounding solvent molecules are forced to hydrogen bond with each other forming cage-like clathrate structures. The resultant decrease in entropy is highly unfavorable. Lipophilic atoms may be sulphurs which are neither polar nor hydrogen acceptors and carbon atoms which are not polar.

Van der Waal's bonds are non-specific forces found between atoms which are 3-4Å apart. They are weaker and less specific than hydrogen and electrostatic bonds. The distribution of electronic charge around an atom changes with time and, at any instant, the charge distribution is not symmetric. This transient asymmetry in electronic charge

induces a similar asymmetry in neighboring atoms. The resultant attractive forces between atoms reaches a maximum at the Van der Waal's contact distance but diminishes very rapidly at about 1Å to about 2Å. Conversely, as atoms become separated by less than the contact distance, increasingly strong repulsive forces become dominant as the outer electron clouds of the atoms overlap. Although the attractive forces are relatively weak compared to electrostatic and hydrogen bonds (about 0.6 Kcal/mol), the repulsive forces in particular may be very important in determining whether a peptide ligand may bind successfully to a protein.

In one embodiment, the Böhm scoring function (SCORE1 approach) is used to estimate the binding constant. (Böhm, H.J., *J. Comput Aided Mol. Des.,* 8(3):243-256 (1994) which is hereby incorporated in its entirety). In another embodiment, the scoring function (SCORE2 approach) is used to estimate the binding affinities as an indicator of a ligand containing a T-cell epitope (Böhm, H.J., *J. Comput Aided Mol. Des.,* 12(4):309-323 (1998) which is hereby incorporated in its entirety). However, the Böhm scoring functions as described in the above references are used to estimate the binding affinity of a ligand to a protein where it is already known that the ligand successfully binds to the protein and the protein/ligand complex has had its structure solved, the solved structure being present in the Protein Data Bank ("PDB"). Therefore, the scoring function has been developed with the benefit of known positive binding data. In order to allow for discrimination between positive and negative binders, a repulsion term must be added to the equation. In addition, a more satisfactory estimate of binding energy is achieved by computing the lipophilic interactions in a pairwise manner rather than using the area based energy term of the above Böhm functions. Therefore, in a preferred embodiment, the binding energy is estimated using a modified Böhm scoring function. In the modified Böhm scoring function, the binding energy between protein and ligand ($\Delta G_{bind}$) is estimated considering the following parameters: The reduction of binding energy due to the overall loss of translational and rotational entropy of the ligand ($\Delta G_0$); contributions from ideal hydrogen bonds ($\Delta G_{hb}$) where at least one partner is neutral; contributions from unperturbed ionic interactions ($\Delta G_{ionic}$); lipophilic interactions between lipophilic

ligand atoms and lipophilic acceptor atoms ($\Delta G_{lipo}$); the loss of binding energy due to the freezing of internal degrees of freedom in the ligand, i.e., the freedom of rotation about each C-C bond is reduced ($\Delta G_{rot}$); the energy of the interaction between the protein and ligand ($E_{VdW}$). Consideration of these terms gives equation 1:

$$(\Delta G_{bind}) = (\Delta G_0) + (\Delta G_{hb} x N_{hb}) + (\Delta G_{ionic} x N_{ionic}) + (\Delta G_{lipo} x N_{lipo}) + (\Delta G_{rot} + N_{rot}) + (E_{VdW}).$$

Where N is the number of qualifying interactions for a specific term and, in one embodiment, $\Delta G_0$, $\Delta G_{hb}$, $\Delta G_{ionic}$, $\Delta G_{lipo}$ and $\Delta G_{rot}$ are constants which are given the values: 5.4, -4.7, -4.7, -0.17, and 1.4, respectively.

The term $N_{hb}$ is calculated according to equation 2:

$$N_{hb} = \sum_{h\text{-}bonds} f(\Delta R, \Delta\alpha)\ x\ f(N_{neighb})\ x\ f_{pcs}$$

$f(\Delta R, \Delta\alpha)$ is a penalty function which accounts for large deviations of hydrogen bonds from ideality and is calculated according to equation 3:

$$f(\Delta R, \Delta\text{-}\alpha) = f1(\Delta R)\ x\ f2(\Delta\alpha)$$

Where: f1 ($\Delta R$) = 1 if $\Delta R$ < = TOL

or =1 - ($\Delta R$ - TOL)/0.4 if $\Delta R$<= 0.4 + TOL
or = 0 i f $\Delta R$>0.4 + TOL

And: f2 ($\Delta\alpha$) = 1 if $\Delta\alpha$ <30°

or = 1- $\Delta\alpha$ - 30) /50 if $\Delta\alpha$ <=80°
or = 0 if $\Delta\alpha$ >80°

TOL is the tolerated deviation in hydrogen bond length = 0.25Å
$\Delta R$ is the deviation of the H-O/N hydrogen bond length from the ideal value = 1.9Å
$\Delta\alpha$ is the deviation of the hydrogen bond angle $\angle_{N/O\text{-}H..O/N}$ from its idealized value of 180°
$f(N_{neighb})$ distinguishes between concave and convex parts of a protein surface and

therefore assigns greater weight to polar interactions found in pockets rather than those found at the protein surface. This function is calculated according to equation 4 below:

$$f(N_{neighb}) = (N_{neighb}/N_{neighb,0})^{\alpha}$$

where $\alpha = 0.5$
$N_{neighb}$ is the number of non-hydrogen protein atoms that are closer than 5Å to any given protein atom.
$N_{neighb,0}$ is a constant = 25
$f_{pcs}$ is a function which allows for the polar contact surface area per hydrogen bond and therefore distinguishes between strong and weak hydrogen bonds and its value is determined according to the following criteria:

$$f_{pcs} = \beta \text{ when } A_{polar}/N_{HB} < 10 \text{ Å}^2$$

$$\text{or } f_{pcs} = 1 \text{ when } A_{polar}/N_{HB} > 10 \text{ Å}^2$$

$A_{polar}$ is the size of the polar protein-ligand contact surface
$N_{HB}$ is the number of hydrogen bonds
$\beta$ is a constant whose value =1.2

For the implementation of the modified Böhm scoring function, the contributions from ionic interactions, $\Delta G_{ionic}$, are computed in a similar fashion to those from hydrogen bonds described above since the same geometry dependency is assumed

The term $N_{lipo}$ is calculated according to equation 5 below:

$$N_{lipo} = \Sigma_{1L} f(r_{1L})$$

$f(r_{IL})$ is calculated for all lipophilic ligand atoms, 1, and all lipophilic protein atoms, L, according to the following criteria:

$$f(r_{1L}) = 1 \text{ when } r_{1L} <= R1 f(r_{1L}) = (r_{1L} - R1)/(R2-R1) \text{ when } R2 < r_{1L} > R1$$

$$f(r_{1L}) = 0 \text{ when } r_{1L} >= R2$$

Where: $R1 = r_1^{vdw} + r_L^{vdw} + 0.5$
and $R2 = R1 + 3.0$
and $r_1^{vdw}$ is the Van der Waal's radius of atom 1
and $r_L^{vdw}$ is the Van der Waal's radius of atom L

The term $N_{rot}$ is the number of rotable bonds of the amino acid side chain and is taken to be the number of acyclic $sp^3$ - $sp^3$ and $sp^3$ - $sp^2$ bonds. Rotations of terminal $-CH_3$ or $- NH_3$ are not taken into account.

The final term, $E_{VdW}$, is calculated according to equation 6 below:

$$E_{vdW} = \varepsilon_1\varepsilon_2((r_1^{vdw} + r_2^{vdw})^{12}/r^{12} - (r_1^{vdw} + r_2^{vdw})^6/r^6),$$

where:

$\varepsilon_1$ and $\varepsilon_2$ are constants dependant upon atom identity
$r_1^{vdw} + r_2^{vdw}$ are the Van der Waal's atomic radii
r is the distance between a pair of atoms.

With regard to Equation 6, in one embodiment, the constants $\varepsilon_1$and $\varepsilon_2$ are given the atom values: C: 0.245, N:

0.283, O: 0.316, S: 0.316, respectively (i.e. for atoms of Carbon, Nitrogen, Oxygen and Sulphur, respectively). With regards to equations 5 and 6, the Van der Waal's radii are given the atom values C: 1.85, N: 1.75, O: 1.60, S: 2.00Å.

It should be understood that all predetermined values and constants given in the equations above are determined within the constraints of current understandings of protein ligand interactions with particular regard to the type of computation being undertaken herein. Therefore, it is possible that, as this scoring function is refined further, these values and constants may change hence any suitable numerical value which gives the desired results in terms of estimating the binding energy of a protein to a ligand may be used and hence fall within the scope of the present invention. As described above, the scoring function is applied to data extracted from the database of side-chain conformations, atom identities, and interatomic distances. For the purposes of the present description, the number of MHC Class II molecules included in this database is 42 models plus four solved structures. It should be apparent from the above descriptions that the modular nature of the construction of the computational method of the present invention means that new models can simply be added and scanned with the peptide backbone library and side-chain conformational search function to create additional data sets which can be processed by the peptide scoring function as described above. This allows for the repertoire of scanned MHC Class II molecules to easily be increased, or structures and associated data to be replaced if data are available to create more accurate models of the existing alleles. The present prediction method can be calibrated against a data set comprising a large number of peptides whose affinity for various MHC Class II molecules has previously been experimentally determined. By comparison of calculated versus experimental data, a cut of value can be determined above which it is known that all experimentally determined T-cell epitopes are correctly predicted. It should be understood that, although the above scoring function is relatively simple compared to some sophisticated methodologies that are available, the calculations are performed extremely rapidly. It should also be understood that the objective is not to calculate the true binding energy *per se* for each peptide docked in the binding groove of a selected MHC Class II protein. The underlying objective is to obtain comparative binding energy data as an aid to predicting the location of T-cell epitopes based on the primary structure (i.e, amino acid sequence) of a selected protein. A relatively high binding energy or a binding energy above a selected threshold value would suggest the presence of a T-cell epitope in the ligand. The ligand may then be subjected to at least one round of amino-acid substitution and the binding energy recalculated. Due to the rapid nature of the calculations, these manipulations of the peptide sequence can be performed interactively within the program's user interface on cost-effectively available computer hardware. Major investment in computer hardware is thus not required. It would be apparent to one skilled in the art that other available software could be used for the same purposes. In particular, more sophisticated software which is capable of docking ligands into protein binding-sites may be used in conjunction with energy minimization. Examples of docking software are: DOCK (Kuntz *et al., J. Mol. Biol.,* 161:269-288 (1982)), LUDI (Böhm, H.J., *J. Comput Aided Mol. Des.,* 8:623-632 (1994)) and FLEXX (Rarey M., *et al.; ISMB,* 3:300-308 (1995)). Examples of molecular modeling and manipulation software include: AMBER (Tripos) and CHARMm (Molecular Simulations Inc.). The use of these computational methods would severely limit the throughput of the method of this invention due to the lengths of processing time required to make the necessary calculations. However, it is feasible that such methods could be used as a 'secondary screen' to obtain more accurate calculations of binding energy for peptides which are found to be 'positive binders' via the method of the present invention. The limitation of processing time for sophisticated molecular mechanic or molecular dynamic calculations is one which is defined both by the design of the software which makes these calculations and the current technology limitations of computer hardware. It may be anticipated that, in the future, with the writing of more efficient code and the continuing increases in speed of computer processors, it may become feasible to make such calculations within a more manageable time-frame. Further information on energy functions applied to macromolecules and consideration of the various interactions that take place within a folded protein structure can be found in: Brooks, B.R., *et al., J. Comput. Chem.,* 4: 187-217 (1983) and further information concerning general protein-ligand interactions can be found in: Dauber-Osguthorpe et al., *Proteins* 4(1):31-47(1988), which are incorporated herein by reference in their entirety. Useful background information can also be found, for example, in Fasman, G.D., ed., *Prediction of Protein Structure and the Principles of Protein Conformation,* Plenum Press, New York, ISBN: 0-306 4313-9.

**SEQUENCE LISTING**

<110> Merck Patent GmbH

<120> METHOD FOR MAPPING AND ELIMINATING T-CELL EPITOPES

<130> P02-088-Bz

<140> PCT/EP03/06110

<141> 2003-06-11

<160> 150

<170> PatentIn version 3.1

<210> 1
<211> 12
<212> PRT
<213> Artificial
<220>
<223> Influenza haemagglutinin peptide sequence
<400> 1

```
Pro Lys Tyr Val Lys Gln Asn Thr Leu Lys Leu Ala
1               5                   10
```

<210> 2
<211> 15
<212> PRT
<213> Artificial
<220>
<223> Chlamydia HSP 60 peptide sequence
<400> 2

```
Lys Val Val Asp Gln Ile Lys Lys Ile Ser Lys Pro Val Gln His
1               5                   10                  15
```

<210> 3
<211> 15
<212> PRT
<213> Artificial
<220>
<223> IFNβ peptide
<400> 3

```
Met Ser Tyr Asn Leu Leu Gly Phe Leu Gln Arg Ser Ser Asn Phe
1               5                   10                  15
```

<210> 4
<211> 15
<212> PRT
<213> Artificial
<220>

## Claims

1. A method for constructing a T-cell epitope map of a subject protein or a fragment thereof by means of peripheral blood mononuclear cells (PBMCs) comprising the following steps:

   (i) antigen priming *in vitro* using the whole subject protein or synthetic peptides representative of and generated from the amino acid sequence of said subject protein or fragments thereof by incubating and culturing the synthetic peptides with PBMC derived T-cells ;
   (ii) treating and culturing the primed T-cells with a cytokine;
   (iii) addition of the primed T-cells to autologous irradiated PBMCs and new priming and culturing with said synthetic peptide antigens, and
   (iv) measuring T-cell response in a T-cell proliferation assay by a pre-selected time course protocol.

2. A method of claim 1, wherein the T-cell comprising PBMCs have been isolated from a multiplicity of different healthy individuals that have not previouslly been exposed to the subject protein, a fragment thereof, or the peptide antigen.

3. A method of claim 2, wherein the PBMCs derive from a pool of donor individuals of sufficient immunological diversity representing more than 90% of the MHC class II repertoire.

4. A method according to claim 1, wherein the T-cell comprising PBMCs have been isolated from individual patients in whom there exists an established immune response to the subject protein or a fragment thereof.

5. A method according to any of the claims 1 - 4, wherein the individual is a human and the subject protein is a human protein.

6. A method of any of the claims 1 - 5, wherein the entire length of the subject protein is scanned for synthetic overlapping peptides of predetermined uniform size and constituted by at least three amino acid residues from the selected region.

7. A method according to claim 6, wherein said synthetic overlapping peptides contain 9 -15 amino acid residues.

8. A method of claim 7, wherein said synthetic peptides contain 15 amino acid residues.

9. A method of any of the claims 1-8, wherein the cytokine is IL-2.

10. A method of any of the claims 1- 9, wherein the time course protocol is carried out according to the following steps:

1. Thaw 1 vial of PBMC per donor
2. Resuspend cells at 2-4x$10^6$ cells/ml in AIM V.
3. Transfer 1 ml to 3 wells of a 24 well plate giving a final concentration of 2-4$\times 10^6$ PBMC/well,
4. Make stock solutions of antigens typically 100$\mu$g/ml for proteins and 2-10 $\mu$M for peptides. Add 1 ml of antigen to each well to give a final concentration 10- 50 $\mu$g/ml protein or 1-5 $\mu$M peptide.
5. Incubate for 5 days.
6. Gently resuspend the cells in the 2 ml cultures by pipetting and from each condition remove 100 $\mu$l cells and place into a round bottom well of a 96 well plate, repeat this three times for each culture condition (total of 300 $\mu$l removed from each culture condition per time point).
7. To each well of cells in the 96 well plate add 1 $\mu$Ci/well 3H [Thy] in 100 $\mu$l AIM V.
8. Incubate overnight and harvest.
9. Repeat stage 6-8 for days 6,7, and 8 (day 9 can be included if necessary).
10. Make SI determinations and plot the SI versus time for each antigen.

11. A method according to any of the claims 1-10, wherein the subject protein is a therapeutic protein.

12. A T-cell activation assay comprising any of the methods of claims 1 - 11.

13. Use of the T-cell activation assay of claim 12 for detecting weakly immunogenic proteins, polypeptides or peptides.

14. A method for preparing an immunogenicly modified biological subject protein from a parental molecule having the same biological activity, wherein the modified molecule has an amino acid sequence different from that of said parent molecule and exhibits a reduced immunogenicity relative to the parent molecule when exposed to the immune system of a given species; said method comprises: (i) determining the amino acid sequence of the parental protein or part thereof; (ii) identifying one or more potential T-cell epitopes, (iii) designing new sequence variants by alteration of at least one amino acid residue within the originally identified T-cell epitope sequences, said variants are modified in such a way to substantially reduce or eliminate the activity or number of the T-cell epitope sequences and / or the number of MHC allotypes able to bind peptides derived from said biological molecule as determined by the binding of the peptides to MHC and the binding of peptide-MHC complexes to T-cells, respectively, (iv) constructing such sequence variants by recombinant DNA techniques and testing said variants in order to identify one or more variants with desirable properties, and (v) optionally repeating steps (ii) - (iv),
**characterized in that** the identification of T-cell epitope sequences according to step (ii) comprises a method according to any of the claims 1-11.

15. A method of claim 14, wherein the identification step(ii) further comprises by means of computational methods calculating MHC Class II molecule binding score for each of said sampled synthetic peptide segments by summing assigned values for each hydrophobic amino acid residue side chain present in said sampled amino acid residue segment, and selecting at least one of said synthetic peptide segments for modification.

**16.** A method of claim 15, wherein the selected synthetic peptide has an immunogenic stimulation index (SI) of more than 2.0, wherein the SI is obtained by division of the T-cell proliferation score measured to the selected peptide by the score measured in cells not contacted with the peptide.

**17.** A method according to any of the claims 14-16, wherein the modified subject protein has an immunogenic stimulation index (SI) of less than 1.8.

**Patentansprüche**

**1.** Verfahren zur Typisierung von T-Zell-Epitopen eines Testproteins oder eines Fragments davon mit Hilfe von mononucleärer Zellen aus peripherem Blut (PBMC), das die folgenden Schritte umfasst:

(i) In-vitro-Antigenpriming unter Verwendung des gesamten Testproteins oder synthetischer Peptide, die für die Aminosäuresequenz des Testproteins oder von Fragmenten davon repräsentativ oder daraus hergestellt sind, durch Inkubieren und Kultivieren der synthetischen Peptide mit aus PBMC stammenden T-Zellen;
(ii) Behandeln und Kultivieren der geprimeten T-Zellen mit einem Cytokin;
(iii) Zugabe der geprimeten T-Zellen zu autologen, bestrahlten PBMC und erneutes Priming und Kultivieren mit den synthetischen Peptidantigenen und
(iv) Messen der T-Zell-Antwort in einem T-Zell-Proliferationsassay durch ein zuvor ausgewähltes Zeitverlaufsprotokoll.

**2.** Verfahren nach Anspruch 1, wobei die T-Zellen umfassenden PBMC aus einer Vielzahl von verschiedenen gesunden Individuen isoliert worden sind, die dem Testprotein, einem Fragment davon oder dem Peptidantigen zuvor nicht ausgesetzt waren.

**3.** Verfahren nach Anspruch 2, wobei die PBMC aus einem Pool von Donorindividuen mit ausreichender immunologischer Diversität stammen, die mehr als 90% des MHC-Klasse-II-Repertoires wiedergeben.

**4.** Verfahren nach Anspruch 1, wobei die T-Zellen umfassenden PBMC aus einzelnen Patienten isoliert worden sind, bei denen eine etablierte Immunantwort auf das Testprotein oder ein Fragment davon besteht.

**5.** Verfahren nach einem der Ansprüche 1-4, wobei das Individuum ein Mensch ist und das Testprotein ein Humanprotein ist.

**6.** Verfahren nach einem der Ansprüche 1-5, wobei die gesamte Länge des Testproteins hinsichtlich synthetischer überlappender Peptide einer vorbestimmten, gleichmäßigen Größe, die von mindestens drei Aminosäureresten aus der ausgewählten Region gebildet werden, untersucht wird.

**7.** Verfahren nach Anspruch 6, wobei die synthetischen überlappenden Peptide 9-15 Aminosäurereste enthalten.

**8.** Verfahren nach Anspruch 7, wobei die synthetischen Peptide 15 Aminosäurereste enthalten.

**9.** Verfahren nach einem der Ansprüche 1-8, wobei es sich bei dem Cytokin um IL-2 handelt.

**10.** Verfahren nach einem der Ansprüche 1-9, wobei das Zeitverlaufsprotokoll gemäß den folgenden Schritten durchgeführt wird:

1. 1 Gläschen PBMC pro Donor auftauen
2. Die Zellen zu 2-4 x $10^6$ Zellen/ml in AIM V resuspendieren
3. 1 ml in 3 Vertiefungen einer Platte mit 24 Vertiefungen überführen, so dass eine Endkonzentration von 2-4 x $10^6$ PBMC/Vertiefung erhalten wird
4. Stammlösungen von Antigenen mit üblicherweise 100 μg/ml für Proteine und 2-10 μM für Peptide herstellen. 1 ml Antigen zu jeder Vertiefung zugeben, so dass eine Endkonzentration von 10-50 μg/ml Protein oder 1-5 μM Peptid erhalten wird
5. 5 Tage inkubieren
6. Die Zellen in den 2-ml-Kulturen vorsichtig durch Pipettieren resuspendieren und von jeder Bedingung 100 μl Zellen entnehmen und in eine Rundbodenvertiefung einer Platte mit 96 Vertiefungen überführen, dreimal für

jede Kulturbedingung wiederholen (insgesamt 300 µl aus jeder Kulturbedingung pro Zeitpunkt entnommen)
7. Zu jeder Vertiefung mit Zellen in der Platte mit 96 Vertiefungen 1 µCi/Vertiefung $^{3}$H-[Thy] in 100 µl AIM V zugeben
8. Über Nacht inkubieren und ernten
9. Stufe 6-8 für die Tage 6, 7 und 8 wiederholen (Tag 9 kann gegebenenfalls hinzugefügt werden)
10. SI-Bestimmungen durchführen und den S1 gegen die Zeit für jedes Antigen auf tragen

**11.** Verfahren nach einem der Ansprüche 1-10, wobei das Testprotein ein therapeutisches Protein ist.

**12.** T-Zell-Aktivierungsassay, der eines der Verfahren der Ansprüche 1-11 umfasst.

**13.** Verwendung des T-Zell-Aktivierungsassays nach Anspruch 12 zum Nachweis schwach immunogener Proteine, Polypeptide oder Peptide.

**14.** Verfahren zur Herstellung eines hinsichtlich seiner Immunogenität modifizierten biologischen Testproteins aus einem Ausgangsmolekül mit der gleichen biologischen Aktivität, wobei das modifizierte Molekül eine andere Aminosäuresequenz als das Ausgangsmolekül besitzt und verglichen mit dem Ausgangsmolekül eine geringere Immunogenität aufweist, wenn es dem Immunsystem einer gegebenen Spezies ausgesetzt wird; wobei das Verfahren die folgenden Schritte umfasst: (i) Bestimmen der Aminosäuresequenz des Ausgangsproteins oder eines Teils davon; (ii) Identifizieren eines oder mehrerer potenzieller T-Zell-Epitope; (iii) Gestalten neuer Sequenzvarianten durch Verändern mindestens eines Aminosäurerestes innerhalb der ursprünglich identifizierten T-Zell-Epitopsequenzen, wobei die Varianten derart modifiziert werden, dass die Aktivität oder Anzahl der T-Zell-Epitopsequenzen und/oder die Anzahl an MHC-Allotypen, die zur Bindung von Peptiden, die von dem biologischen Molekül stammen, wie durch die Bindung der Peptide an MHC bzw. die Bindung von Peptid-MHC-Komplexen an T-Zellen nachgewiesen, in der Lage sind, erheblich verringert oder beseitigt wird, (iv) Konstruieren solcher Sequenzvarianten durch DNA-Rekombinationstechniken und Testen der Varianten, um eine oder mehrere Varianten mit wünschenswerten Eigenschaften zu identifizieren, und (v) gegebenenfalls Wiederholen der Schritte (ii)-(iv), **dadurch gekennzeichnet, dass** die Identifizierung von T-Zell-Epitopsequenzen gemäß Schritt (ii) ein Verfahren nach einem der Ansprüche 1-11 umfasst.

**15.** Verfahren nach Anspruch 14, wobei der Identifizierungsschritt (ii) ferner das Berechnen eines MHC-Klasse-II-Molekül-Bindungswertes mithilfe eines Computerverfahrens für jedes der gesammelten synthetischen Peptidsegmente durch Summieren zugewiesener Werte für jede in dem gesammelten Aminosäurerestsegment vorhandene hydrophobe Aminosäurerestseitenkette und Auswählen mindestens eines der synthetischen Peptidsegmente für eine Modifikation umfasst.

**16.** Verfahren nach Anspruch 15, wobei das ausgewählte synthetische Peptid einen immunogenen Stimulationsindex (SI) von mehr als 2,0 besitzt, wobei der SI durch Division des für das ausgewählte Peptid gemessenen T-Zell-Proliferationswertes durch den in Zellen ohne Kontakt zu dem Peptid gemessenen Wert erhalten wird.

**17.** Verfahren nach einem der Ansprüche 14-16, wobei das modifizierte Testprotein einen immunogenen Stimulationsindex (SI) von weniger als 1,8 besitzt.

## Revendications

**1.** Procédé pour construire une carte d'épitopes de cellules T d'une protéine sujet ou d'un fragment afférent au moyen de cellules mononucléaires du sang périphérique (CMSP), comprenant les étapes qui suivent :

(i) amorçage d'antigène in vitro utilisant la protéine sujet complète ou des peptides synthétiques qui sont représentatifs de la séquence d'acides aminés de ladite protéine sujet ou desdits fragments afférents et qui sont générés à partir de cette même séquence, en incubant et en cultivant les peptides synthétiques avec des cellules T dérivées de CMSP ;
(ii) traitement et culture des cellules T amorcées avec une cytokine ;
(iii) addition des cellules T amorcées à des CMSP irradiées autologues et amorçage et culture nouvellement avec lesdits antigènes de peptide synthétique ; et
(iv) mesure d'une réponse de cellule T au niveau d'un test de prolifération de cellules T au moyen d'un protocole à décours temporel présélectionné.

**2.** Procédé selon la revendication 1, dans lequel les CMSP comprenant des cellules T ont été isolées à partir d'une multiplicité de différents individus sains qui n'ont pas été au préalable exposés à la protéine sujet, à un fragment afférent ou à l'antigène de peptide.

**3.** Procédé selon la revendication 2, dans lequel les CMSP sont dérivées à partir d'un ensemble d'individus donneurs d'une diversité immunologique suffisante représentant plus de 90% du répertoire de classe II de CMH.

**4.** Procédé selon la revendication 1, dans lequel les CMSP comprenant des cellules T ont été isolées à partir de patients individuels au niveau desquels il existe une réponse immunitaire établie vis-à-vis de la protéine sujet ou d'un fragment afférent.

**5.** Procédé selon l'une quelconque des revendications 1 à 4, dans lequel l'individu est un être humain et la protéine sujet est une protéine humaine.

**6.** Procédé selon l'une quelconque des revendications 1 à 5, dans lequel la longueur totale de la protéine sujet est balayée quant à des peptides en chevauchement synthétiques d'une dimension uniforme prédéterminée et constitués par au moins trois résidus d'acide aminé en provenance de la région sélectionnée.

**7.** Procédé selon la revendication 6, dans lequel lesdits peptides en chevauchement synthétiques contiennent des résidus d'acide aminé 9-15.

**8.** Procédé selon la revendication 7, dans lequel lesdits peptides synthétiques contiennent 15 résidus d'acide aminé.

**9.** Procédé selon l'une quelconque des revendications 1 à 8, dans lequel la cytokine est IL-2.

**10.** Procédé selon l'une quelconque des revendications 1 à 9, dans lequel le protocole à décours temporel est mis en oeuvre conformément aux étapes qui suivent :

1. dégel d'un flacon de CMSP par donneur ;
2. resuspension des cellules selon 2-4 x $10^6$ cellules/ml dans AIM V ;
3. transfert de 1 ml sur 3 puits d'une plaque à 24 puits en obtenant une concentration finale de 2-4 x $10^6$ CMSP/puits ;
4. constitution de solutions de stock d'antigènes de typiquement 100 $\mu$g/ml pour les protéines et de typiquement 2-10 $\mu$M pour les peptides, addition de 1 ml d'antigène à chaque puits afin d'obtenir une concentration finale en protéines de 10-50 $\mu$g/ml ou en peptides de 1-5 $\mu$M;
5. incubation pendant 5 jours ;
6. resuspension en douceur des cellules dans les cultures à 2 ml par pipetage et à partir de chaque condition, enlèvement de 100 $\mu$l de cellules et placement dans un puits à fond rond d'une plaque à 96 puits, répétition de ceci trois fois pour chaque condition de culture (un total de 300 $\mu$l est enlevé à partir de chaque condition de culture par point temporel) ;
7. sur chaque puits de cellules dans la plaque à 96 puits, addition de 1 $\mu$Ci/puits 3H [Thy] dans 100 $\mu$l de AIM V ;
8. incubation toute la nuit et récupération ;
9. répétition des étapes 6 à 8 aux jours 6, 7 et 8 (le jour 9 peut être inclus si nécessaire) ; et
10. réalisation de déterminations de SI et tracé du SI en fonction du temps pour chaque antigène.

**11.** Procédé selon l'une quelconque des revendications 1 à 10, dans lequel la protéine sujet est une protéine thérapeutique.

**12.** Test d'activation de cellules T comprenant l'un quelconque des procédés des revendications 1 à 11.

**13.** Utilisation du test d'activation de cellules T de la revendication 12 pour détecter des protéines, des polypeptides ou des peptides faiblement immunogènes.

**14.** Procédé pour la préparation d'une protéine sujet biologique modifiée immuno-géniquement à partir d'une molécule parentale présentant la même activité biologique, dans lequel la molécule modifiée comporte une séquence d'acides aminés qui est différente de celle de ladite molécule de parent et présente une immunogénicité réduite par rapport à la molécule de parent lorsqu'elle exposée au système immunitaire d'une espèce donnée ; ledit procédé comprend : (i) la détermination de la séquence d'acides aminés de la protéine parentale ou d'une partie de celle-ci ; (ii) l'identification d'un ou de plusieurs épitopes de cellules T potentiels ; (iii) la désignation de nouveaux variants de séquence par altération d'au moins un résidu d'acide aminé à l'intérieur des séquences d'épitopes de cellules T identifiées originellement, lesdits variants sont modifiés de manière à réduire de façon substantielle ou à éliminer l'activité ou le nombre des séquences d'épitopes de cellules T et/ou le nombre d'allotypes de CMH pouvant se lier sur des peptides dérivés à partir de ladite molécule biologique comme déterminé par la liaison des peptides sur CMH et par la liaison de complexes peptides-CMH sur des cellules T, de façon respective ; (iv) la construction de ces variants de séquence en recombinant des techniques ADN et en testant lesdits variants afin d'identifier un ou plusieurs variants présentant des propriétés souhaitables et (v) en option, la répétition des étapes (ii) à (iv), **caractérisé en ce que** l'identification de séquences d'épitopes de cellules T conformément à l'étape (ii) comprend un procédé selon l'une quelconque des revendications 1 à 11.

**15.** Procédé selon la revendication 14, dans lequel l'étape d'identification (ii) comprend en outre, au moyen de procédés de calcul, le calcul d'un score de liaison de molécules de classe II de CMH pour chacun desdits segments de peptides synthétiques échantillonnés en sommant des valeurs assignées pour chaque chaîne latérale de résidu d'acide aminé hydrophobe qui est présente dans ledit segment de résidu d'acide aminé échantillonné, et la sélection d'au moins l'un desdits segments de peptides synthétiques pour une modification.

**16.** Procédé selon la revendication 15, dans lequel le peptide synthétique sélectionné présente un indice de stimulation (SI) immunogène supérieur à 2,0, dans lequel le SI est obtenu par division du score de prolifération de cellules T comme mesuré sur le peptide sélectionné par le score mesuré dans des cellules non en contact avec le peptide.

**17.** Procédé selon l'une quelconque des revendications 14 à 16, dans lequel la protéine sujet modifiée présente un indice de stimulation (SI) immunogène inférieur à 1,8.

# FIGURE 1

## INFβ IMMUNOGENIC REGIONS

## REGION 1 (R1)

```
Residue#   49                      72
            QFQKEDAALTIYEMLQNIFAIFRQ

Stimulating
Peptides:
     #17    QFQKEDAALTIYEML
     #18       KEDAALTIYEMLQNI
     #19          AALTIYEMLQNIFAI
     #20             TIYEMLQNIFAIFRQ
```

## REGION 2 (R2)

```
Residue #  124                   145
           RYYGRILHYLKAKEYSHCAWT

Stimulating
Peptides:
     #42   RYYGRILHYLKA
     #43      GRILHYLKAKEY
     #44         LHYLKAKEYSHC
     #45            LKAKEYSHCAWT
```

# FIGURE 2

## IFNα IMMUNOGENIC REGIONS

## REGION 1 (R1)

```
Residue#    24                              57
            ISLFSCLKDRHDFGFPQEEFGNQFQKAETIPVLH

Stimulating
Peptides:
     #9      SLFSCLKDRHDFGFP
     #10        SCLKDRHDFGFPQEE
     #11           KDRHDFGFPQEEFGN
     #12              HDFGFPQEEFGNQFQ
     #13                 GFPQEEFGNQFQKAE
     #14                    QEEFGNQFQKAETIP
     #15                       FGNQFQKAETIPVLH
```

## REGION 2 (R2)

```
Residue #     64            78
              FNLFSTKDSSAAWDE

Stimulating
Peptides:
     #21    QQIFNLFSTKDSSAA
     #22       FNLFSTKDSSAAWDE
     #23          FSTKDSSAAWDETLL
     #24             KDSSAAWDETLLDKF
```

## REGION 3 (R3)

```
Residue #     110                 129
              LMKEDSILAVRKYFQRITLY

Stimulating
Peptides:
     #36 TETPLMKEDSILAVR
     #37    PLMKEDSILAVRKYF
     #38       KEDSILAVRKYFQRI
     #39          SILAVRKYFQRITLY
     #40             AVRKYFQRITLYLKE
```

FIGURE 3

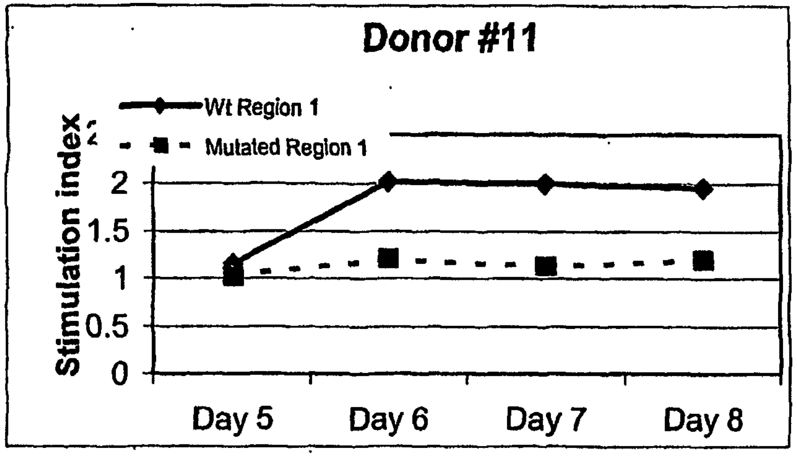


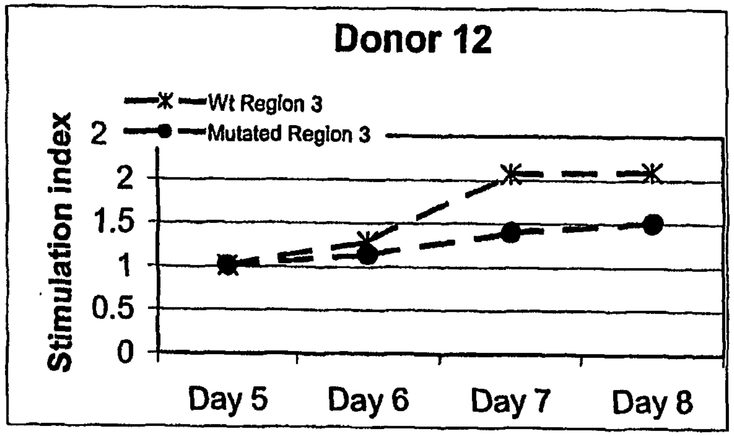


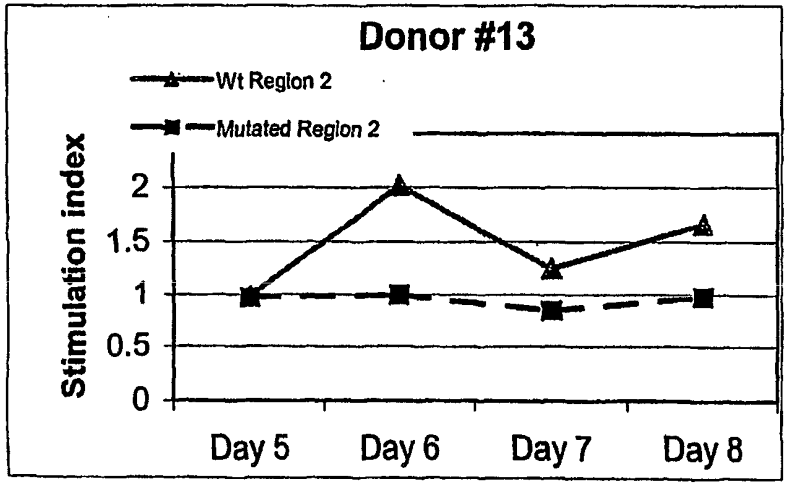


| IFN α PEPTIDE | PEPTIDE SEQUENCE |
|---|---|
| Region 1 | QMRRISLFSCLKDRHDFGFP |
| Mutated Region 1 | QMRRQSLFSCLKDRHDFGFP |
| Region 2 | EMIQQIFNLFSTKDSSAAWDETLLDKFY |
| Mutated Region 2 | EMTQQIANLFSTKDSSAAHDETLLDKFY |
| Region 3 | TPLMKEDSILAVRKYFQRITLYLKEKKYSPCAW |
| Mutated Region 3 | TPLMKEDSRLAVRKYFQRITNYLKEKKYSPCAW |